(19) Europäisches Patentamt
European Patent Office
Office européen des brevets

(11) **EP 2 843 415 B1**

(12) **EUROPEAN PATENT SPECIFICATION**

(45) Date of publication and mention
of the grant of the patent:
**01.04.2020 Bulletin 2020/14**

(51) Int Cl.:
***G01N 33/68*** (2006.01)

(21) Application number: **13182392.4**

(22) Date of filing: **30.08.2013**

(54) **Methods for cardiovascular risk assessment in diabetes patients**

Verfahren für die Beurteilung des kardiovaskulären Risikos bei Diabetikern

Procédés pour l'évaluation d'un risque cardiovasculaire chez des patients atteints de diabète

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**04.03.2015 Bulletin 2015/10**

(73) Proprietor: **LURIC Datenbank GbR
68165 Mannheim (DE)**

(72) Inventors:
• **Niessner, Alexander
1180 Wien (AT)**
• **Goliasch, Georg
1060 Wien (AT)**
• **Kleber, Marcus E.
69412 Eberbach (DE)**

• **März, Winfried
69493 Hirschberg/Leutershausen (DE)**

(74) Representative: **Nottrott, Stephanie
Patentanwälte
Isenbruck Bösl Hörschler PartG mbB
Prinzregentenstraße 68
81675 München (DE)**

(56) References cited:
**WO-A2-2012/174282     US-A1- 2006 019 315**

• **R. B. SCHNABEL ET AL: "Multiple marker
approach to risk stratification in patients with
stable coronary artery disease", EUROPEAN
HEART JOURNAL, vol. 31, no. 24, 2 December
2010 (2010-12-02), pages 3024-3031, XP55038381,
ISSN: 0195-668X, DOI: 10.1093/eurheartj/ehq322**

**Description**

The present invention relates to the field of laboratory diagnostics. Specifically, the present invention relates to methods for determining the risk of mortality in a patient with diabetes mellitus by investigating biomarkers from various pathophysiological pathways. These methods allow for the reduction of the patient's risk of mortality by providing suitable and improved medical treatments to the patient.

An aim of modern medicine is to provide personalized or individualized treatment regimens. Those are treatment regimens which take into account a patient's individual needs or risks. Individualized treatment regimens offer benefits for the individual patient as well as for society as a whole. For the individual patient personalized treatment avoids excessive therapy while ensuring that necessary measures are taken. As every therapy may cause undesired harmful side effects, the avoidance of unnecessary therapies saves the patient from potentially harmful side effects. On the other hand, the identification of patients with special needs ensures that these individuals receive the appropriate treatment. For the health system as a whole the avoidance of unnecessary therapies allows for a more economic use of resources.

Individualized treatment regimens require appropriate diagnostic tools in order to separate those patients who benefit from certain therapeutic measures from the patients who do not. Therefore, the development of individualized treatment regimens critically depends on the development of novel diagnostic tools and procedures. Because the prevention of future disease is often more effective than the therapy of already existing disease, diagnostic tools and methods for risk stratification with respect to future diseases are especially desirable.

There is broad evidence that diabetes mellitus is associated with elevated all-cause and cardiovascular mortality. So far, cardiovascular risk assessment in patients with diabetes mellitus relied on traditional cardiovascular risk factors [7]. However, numerous novel biomarkers have been found to be independent predictors of cardiovascular disease, which might significantly improve risk prediction in diabetic patients. Cardiovascular diseases are amongst the major causes of death in industrialized countries and often a cause of death in patients suffering from diabetes. However, despite highly effective measures to control conventional risk factors the incidence of cardiovascular events remains high. This highlights the need to identify independent non-conventional risk factors for calculating and determining the risk of mortality in diabetic patients.

US 2006/0019315 relates to the risk stratification of co-morbidities, in particular cardiovascular complications, in diabetes patients. Thereby, US 2006/0019315 pertains to a method for diagnosing whether a diabetes patient is suffering from a cardiovascular complication or is at risk of suffering from a cardiovascular complication, comprising the steps of measuring the level of a cardiac hormone and diagnosing the cardiovascular complication or the risk of a cardiovascular complication by comparing the measured level to a known level associated with the cardiovascular complication or the risk.

Schnabel et al., 2010, Eur Heart J, 31: 3024-3031 relates to a multiple marker approach to risk stratification in patients with stable coronary artery disease. Thereby 12 biomarkers were investigated separately and the top biomarkers were also investigated in combination. The authors concluded from their investigations that the combined measurement did not improve risk stratification compared with the strongest single biomarkers.

Therefore, there is always a need for providing improved methods of risk prediction in cardiovascular medicine including improved methods of cardiovascular risk assessment in patients with diabetes.

In a first aspect, the present invention provides a method for determining the risk of mortality in a patient with diabetes mellitus, comprising the steps of

a) determining a set of biological parameters obtained from the patient, wherein the set of biological parameters comprises the following variables:

the patient's gender,

the patient's age,

at least one indicator of myocardial performance, wherein the at least one indicator of myocardial performance is defined by the patient's blood concentration of one or more natriuretic peptide(s)) selected from atrial natriuretic peptide (ANP) and brain natriuretic peptide (BNP) such as pre-proANP, proANP, N-terminal pro-ANP (Nt-proANP), mid-regional-pro-atrial natriuretic peptide (MR-proANP), ANP, pre-proBNP, proBNP, N-terminal pro-B-type natriuretic peptide (Nt-proBNP), or BNP,

an indicator of renal performance, wherein the indicator of renal performance is defined by the patient's blood concentration of renin,

the duration of diabetes mellitus in the patient defined in years,

an indicator of lipid metabolism, wherein the indicator of lipid metabolism is defined by the patient's blood activity of lipoprotein associated phospholipase A2 (Lp-PLA2), and

an indicator of blood calcium and/or phosphate metabolism, wherein the indicator of blood calcium and/or phosphate metabolism is defined by the patient's blood concentration of vitamin $D_3$;

b) setting the biological parameter into correlation, thereby providing a risk score, wherein the risk score is indicative for all-cause mortality of the patient within the next years, and wherein the risk score is indicative for a medical treatment of the patient to reduce the patient's risk of all-cause mortality,

further characterized in that the risk score is a simplified risk score $Y_1$, and in that the biological parameter are set into correlation by a sum of seven quantities, the sum thereby providing the simplified risk score $Y_1$,

wherein a first quantity is equal to 1 if the patient's gender is male and otherwise equal to 0,

wherein a second quantity is equal to 1 if the patient's age is equal to or more than 75 years and otherwise equal to 0,

wherein a third quantity is equal to 1 if the concentration of the at least one natriuretic peptide is equal to or more than 400 ng/L, equal to 2 if the concentration of the at least one natriuretic peptide is equal to or more than 2000 ng/L and otherwise equal to 0,

wherein a fourth quantity is equal to 1 if the duration of diabetes mellitus is equal to or more than 5 years and otherwise equal to 0,

wherein a fifth quantity is equal to 1 if the concentration of renin is equal to or more than 50 pg/mL and otherwise equal to 0,

wherein a sixth quantity is equal to 1 if the activity of lipoprotein associated phospholipase A2 (Lp-PLA2) is higher than 450 U/L and otherwise equal to 0,

wherein a seventh quantity is equal to 1 if the concentration of vitamin $D_3$ is equal to or less than 10 ng/L and otherwise equal to 0.

Disclosed is a method for determining the risk of mortality in a patient with diabetes mellitus, comprising the steps of

a) analysing a set of biological parameters obtained from the patient, wherein the set of biological parameters comprises the following variables:

the patient's gender,
the patient's age,
at least one indicator of myocardial performance,
an indicator of renal performance,
the duration of diabetes mellitus in the patient defined in years,
an indicator of lipid metabolism, and
an indicator of blood calcium and/or phosphate metabolism,

b) setting the biological parameter into correlation, thereby providing a risk score, wherein the risk score is indicative for all-cause mortality of the patient within the next years, and wherein the risk score is indicative for a medical treatment of the patient to reduce the patient's risk of all-cause mortality.

The term "determining the risk of mortality" as used herein refers to assessing the probability according to which a subject will die within a certain time window, i.e. the predictive window. In accordance with the present invention, the predictive window is preferably within 3 years, 5 years, 8 years, or 10 years or more after determination of the risk of mortality. However, as will be understood by those skilled in the art, such an assessment is usually not intended to be correct for 100 % of the subjects to be investigated. The term, however, requires that prediction can be made for a statistically significant portion of subjects in a proper and correct manner. Whether a portion is statistically significant can be determined without further ado by the person skilled in the art using various well known statistic evaluation tools, e.g., the Mann-Whitney-U test, Cox proportional hazard regression analysis, Pearson correlation and Harrell's C-statistic. Details are, for example, found in Dowdy and Wearden, Statistics for Research, John Wiley & Sons, New York 1983. Continuous data are presented as median and interquartile range, discrete data as counts and percentages. Continuous variables preferably undergo log transformation before entering analysis. Preferred confidence intervals are at least 95 %, at least 97 %, at least 98 % or at least 99 %. The p-values are, preferably, 0.005, or 0.0001. The discriminatory power of the designed risk score is assessed using Harrell's C-statistic. The improvement of individual risk prediction is preferably examined by net reclassification improvement. Preferably, the probability envisaged by the present invention allows that the prediction of an increased, normal or decreased risk will be correct for at least 60 %, at least 70 %, at least 80 %, or at least 90 %, and preferably of at least 95 % of the subjects of a given cohort or population. Determining the risk of mortality preferably relates to determining whether or not there is an increased risk of mortality compared to the average risk of mortality in a population of subjects rather than giving a precise probability for the said risk.

The term "risk score" as used herein generally means an indicator for the risk of mortality with respect to a certain period of time. In particular, the risk score of the present invention provides an estimate of how long the patient is expected to survive a certain period of time, or alternatively, of how likely it is that the patient will die within this time window. Therefore, in the context of the present invention, the risk score can either be seen as an indicator of a particular survival or mortality rate. In the first aspect of the present invention, the risk score is a simplified risk score $Y_1$.

The method of the present invention is, preferably, an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison in step (b).

In the context of the present invention, the risk score is indicative for all-cause mortality of the patient. The term "all-cause mortality" generally refers to all cased of death due to any cause.

In a preferred embodiment, the risk score is indicative for cardiovascular mortality.

The term "cardiovascular mortality" generally means all cases of death caused and/or related to cardiovascular diseases, preferably caused by diseases of the arterial tree such as, for example, atherosclerosis and/or thrombosis.

The term "cardiovascular disease" as used herein generally refers to all kind of diseases that involve the heart or blood vessels. Cardiovascular disease further refers to any disease that affects the cardiovascular system (as described, for example, in the International Statistical Classification of Diseases and Related Health Problems 10th Revision (ICD10), ICD-10 Chapter IX: Diseases of the circulatory system). The causes of cardiovascular disease are diverse but atherosclerosis and/or hypertension are the most common. Death due to cardiovascular diseases may comprise myocardial infarction, death from congestive heart failure, death from stroke, death following coronary artery bypass graft surgery or death following percutaneous coronary intervention.

In a preferred embodiment, cardiovascular mortality is caused by a disease of the arterial tree, preferably by atherosclerosis and/or thrombosis.

The term "patient" as used herein generally refers to a mammal, preferably to a human.

The term "biological parameter" as used in the context of the present invention generally includes all kind of gene expression products which are differentially expressed, i.e. up regulated or down regulated in presence or absence of a certain condition, disease, or physical situation. A biological parameter of the present invention is also referred to as a biomarker. A biological parameter according to the present invention may also mean a biological parameter of form of the patient's age, the patient's gender and/or the patient's heart rate. Equally preferred is that a biological parameter according to the present invention may be in form of a protein product, preferably in form of an enzyme, a nucleic acid (including mRNA), a peptide, or a small molecule compound, in particular in form of its concentration or amount. The amount of a suitable biological parameter can indicate the presence or absence of the condition, disease, or a physical state, and thus allow for a particular diagnosis. For example, biological parameters reflecting myocardial performance, inflammation, lipid metabolism, renal performance and/or cardiovascular function are well known in the art including their analysis by means of commercially available routine assay systems and antibodies (see, for example, Schnabel et al., 2010. Eur Heart J, 31: 3024-3031). In the context of the present invention, the set of biological parameters determined in the method of the present invention includes those parameters, as identified above under the first aspect of the present invention.

The term "indicator of myocardial performance" as used in the context of the present invention generally refers to any biological parameter which provides information about the medical condition of the patient's heart. In particular, an indicator of mycocardial performance means a biological parameter that provides information about the patient's cardiovascular function. Examples of biological parameters which indicate the patient's myocardial performance include, but are not limited to, copeptin, C-terminal-pro-endothelin-1, mid-regional-pro-adrenomedullin (MR-proADM), mid-regional-pro-atrial natriuretic peptide (MR-proANP), and N-terminal-pro-B-type natriuretic peptide (Nt-proBNP). An indicator of myocardial performance according to the present invention may also refer to the patient's left ventricular ejection fraction (LVEF) which is a measurement of the percentage of blood leaving the patient's heart each time it contracts.

In the first aspect of the present invention, the "indicator of myocardial performance" according to the present invention is defined by the patient's blood concentration of natriuretic peptides. The term "natriuretic peptides" generally refers all known atrial natriuretic peptides (ANP) and brain natriuretic peptides (BNP) described in the art (Bonow 1996, Circulation 93, 1946), including, for example, pre-proANP, proANP, NT-proANP, ANP and pre-proBNP, proBNP, NT-proBNP, and BNP. The pre-pro peptide (134 amino acids in the case of pre-proBNP) comprises a short signal peptide, which is enzymatically cleaved releasing the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferably, natriuretic peptides

EP 2 843 415 B1

according to the present invention are NT-proANP, ANP, and, more preferably, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol 167, 239). Preanalytics are more robust with NT-proBNP allowing easy transportation of the sample to a central laboratory (Mueller 2004, Clin Chem Lab Med 42, 942). Blood samples can be stored at room temperature for several days or may be mailed or shipped without recovery loss. In contrast, storage of BNP for 48 hours at room temperature or at 4° Celsius leads to a concentration loss of at least 20 % (Wu 2004, Clin Chem 50, 867). Therefore, depending on the time-course or properties of interest, either measurement of the active or the inactive forms of the natriuretic peptide can be advantageous. Preferred natriuretic peptides according to the present invention are NT-proBNP or variants thereof. The human NT-proBNP, is a polypeptide comprising, preferably, 76 amino acids in length corresponding to the N-terminal portion of the human NT-proBNP molecule. An alternatively preferred natriuretic peptide is the mid-regional-pro-atrial natriuretic peptide (MR-proANP). Methods for measurement natriuretic peptides have been described (Ala-Kopsala 2004, Clin Chem 50, 1576), and the concentration of the natriuretic peptides is preferably calculated in pg/ml, but may also be indicated by any other suitable concentration format, such as, for example, pmol/dl. Preferably, the natriuretic peptide is N-terminal pro-B-type natriuretic peptide (Nt-proBNP).

The term "indicator of renal performance" as used herein generally means any biological parameter which provides information about the medical condition of the patient's kidney(s). In particular, an indicator of renal function or performance is a biological parameter or a biomarker that provides information about the renal function. Examples of biological parameters include, but are not limited to, cystatin C, serum creatinine, and renin (Schnabel et al., 2010. Eur Heart J; 31: 2024-3031). In the first aspect of the present invention, the indicator of renal performance is defined by the patient's blood concentration of renin.

Generally, the indicator of renal function may be defined by the patient's blood concentration of serum creatinine. Serum creatinine is the most commonly used indicator of renal function. The concentration of serum creatinine can be measured in a blood sample by standard methods, such as the Jaffe routine method. The concentration of serum creatinine is preferably calculated in mg/dl, but may be indicated by any other suitable concentration format, such as, for example, pmol/dl.

Alternatively, the indicator of renal performance may also be defined by the patient's blood concentration of cystatin C and/or renin. Cystatin C, also referred to as cystatin 3 (and formerly known as gamma trace, post-gamma-globulin or neuroendocrine basic polypeptide) is a protein encoded by the CST3 gene. Renin is an enzyme produced by the kidney. Cystatin C and renin are routinely used biomarkers for evaluating the kidney function and/or performance of a patient. The concentration of cystatin C in serum can be defined by standard methods known in the art and is preferably measured, calculated and indicated in mg/L. The same applies for the concentration of renin in a patient's blood sample, the concentration of which is preferably indicated in pg/mL, $\mu$g/mL, or equivalently in mU/L, or U/L

Alternatively, the indicator of renal performance may also be an indicator of blood pressure regulation.

The term "blood sample" as referred to herein preferably refers to a sample of blood which has been obtained from the patient by standard routine practice. It the context of the present invention, however, it is also included that the biological parameter(s) of interest is/are determined by the use of any other sample of the patient as input material, such as, for example, a sample of a body fluid, a sample of separated cells or a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which produce the marker referred to herein.

The patient's gender may be male or female.

The patient's age is defined by the patient's age in years.

The term "duration of diabetes mellitus in the patient defined by years" generally refers to the period of time in years since which the patients suffers from diabetes mellitus. In particular, this period of time may refer to the time point when the diabetes mellitus was first diagnosed in the patient. Alternatively, the duration of diabetes mellitus may indicate the point of time when the patient was first treated to cure one or more of the diabetes mellitus symptom(s), and/or the time point when the patient was first set on medication with insulin.

The term "indicator of lipid metabolism" generally means any parameter indicating lipid metabolism in the patient's body, including, but not limited to, the concentration and/or level of particular blood lipids. In the first aspect of the present invention, the indicator of lipid metabolism is defined by the patient's blood activity of lipoprotein associated phospholipase A2 (Lp-PLA2), preferably indicated in U/L.

Generally, the indicator of lipid metabolism may also be an indicator of subclinical inflammation and/or an indicator of atherosclerotic plaque stability.

In the first aspect of the present invention, the indicator of blood calcium and/or phosphate metabolism is defined by the patient's blood concentration of vitamin $D_3$ including 25(OH) vitamin $D_3$ and 1.23(OH) vitamin $D_3$ (active form). The term "indicator of blood calcium and/or phosphate metabolism" as used herein generally means any biological parameter which is indicative for or related to the concentration or the level of calcium and/or phosphate, preferably calcium and/or phosphate ions, in the patient's blood, preferably in the patient's blood serum. Examples of an indicator of blood calcium and/or phosphate metabolism include vitamin D (also known as cholecalciferol or calciol), vitamin $D_3$, 25(OH) vitamin $D_3$, 1.23(OH) vitamin $D_3$ (active form), or parathormone (parathyroid hormone, PTH), including all kinds of metabolism substances related to the vitamin D pathway.

In a particularly preferred embodiment, the indicator of blood calcium metabolism and/or the indicator of phosphate metabolism is 25(OH) vitamin $D_3$.

Disclosed is a method for determining the risk of mortality in a patient with diabetes mellitus, comprising the steps of

a) analysing a set of biological parameters obtained from the patient, wherein the set of biological parameters comprises the following variables:

the patient's gender,
the patient's age,
at least one indicator of myocardial performance,
an indicator of renal performance,
the duration of diabetes mellitus in the patient defined in years,
an indicator of lipid metabolism,
and an indicator of blood calcium and/or phosphate metabolism,

b) setting the biological parameter into correlation, thereby providing a risk score, wherein the risk score is indicative for all-cause mortality of the patient within the next years, and wherein the risk score is indicative for a medical treatment of the patient to reduce the patient's risk of all-cause mortality,

wherein

i) the at least one indicator of myocardial performance is defined by the patient's blood concentration of one or more natriuretic peptide(s), preferably by the patient's blood concentration of N-terminal pro-B-type natriuretic peptide (Nt-proBNP),

ii) the indicator of renal performance is defined by the patient's blood concentration of renin,

iii) the indicator of lipid metabolism is defined by the patient's blood activity of lipoprotein associated phospholipase A2 (Lp-PLA2), and

iv) the indicator of blood calcium and/or phosphate metabolism is defined by the patient's blood concentration of vitamin $D_3$, preferably by the patient's blood concentration of 25(OH)-vitamin $D_3$.

In the first aspect of the method of the invention, the risk score of the invention is a simplified risk simplified risk score $Y_1$, wherein the biological parameter are set into correlation by a sum of seven quantities, the sum thereby providing the simplified risk score $Y_1$,
wherein a first quantity is equal to 1 if the patient's gender is male and otherwise equal to 0,
wherein a second quantity is equal to 1 if the patient's age is equal to or more than 75 years and otherwise equal to 0,
wherein a third quantity is equal to 1 if the concentration of one or more natriuretic peptide(s) is equal to or more than 400 ng/L, equal to 2 if the concentration of natriuretic peptide is equal to or more than 2000 ng/L and otherwise equal to 0, wherein the natriuretic peptide is preferably N-terminal pro-B-type natriuretic peptide (Nt-proBNP),
wherein a fourth quantity is equal to 1 if the duration of diabetes mellitus is equal to or more than 5 years and otherwise equal to 0,
wherein a fifth quantity is equal to 1 if the concentration of renin is equal to or more than 50 pg/mL and otherwise equal to 0,
wherein a sixth quantity is equal to 1 if the concentration of vitamin $D_3$, preferably the concentration of 25(OH)-vitamin $D_3$, is equal to or less than 10 ng/L and otherwise equal to 0,
wherein a seventh quantity is equal to 1 if the activity of lipoprotein associated phospholipase A2 (Lp-PLA2) is higher than 450 U/L and otherwise equal to 0.

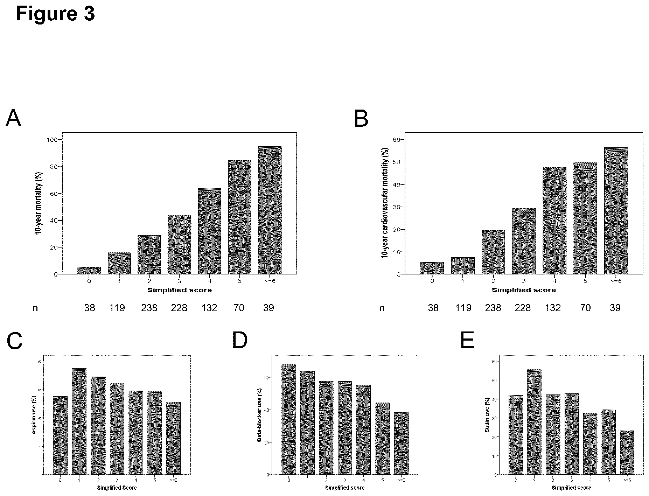

The simplified risk score $Y_1$ of the present invention has been designed for optimal clinical use and its calculation is exemplified in detail by the Example section of the present application, in particular by the results of Figure 3. In the context of the present invention, it has surprisingly been found that the simplified risk score $Y_1$ has an excellent discriminatory power for 10-year survival with a C-statistic of 0.72 (P< 0.001) for all-cause mortality and for 10-years survival free of cardiovascular death with a value of 0.72 (P< 0.001). The discriminatory ability of the risk score of the present invention is, therefore, superior to the one known in the art [7].

In the context of the present invention, is has further been found that a particular risk score can be correlated with a particular risk class of mortality, preferably with a class of low risk of mortality, a class of intermediate (or moderate) risk of mortality and a class of high risk of mortality.

This classification is applicable for all mortality risk scores, in particular for the risk scores $Y_1$, the classification of which is further exemplified by the Examples of the present invention.

Using the simplified risk score $Y_1$, the risk of mortality was calculated by dividing the number of patients who died within a certain time window, in particular within the time window of 10 years, by the number of patients which had initially been classified with this risk score. Thereby, different risk scores could be correlated with different risks of mortality, in particular with different risk score classes as exemplified in the Examples of the present invention.

Therefore, according to a preferred embodiment of the present invention, a simplified risk score $Y_1$ of equal to 0 or equal to 1 indicates a low risk of mortality, while a simplified risk score $Y_1$ of equal to 2 or equal to 3 indicates an moderate risk of mortality, and a simplified risk score $Y_1$ of equal to or more than 4 indicates a high risk of mortality.

Here, a low risk of mortality preferably means a risk of mortality of equal to or below 20 %, an intermediate risk of mortality means a risk of mortality of equal to or below 40 %, while a high risk of mortality means a risk of mortality of at least 50 %, preferably of between 50 % and 60 %.

Preferably, the simplified risk score $Y_1$ is inversely associated with the use of acetylsalicylic acid, beta blocker(s) and/or inhibitors of cholesterol synthesis, preferably CSE inhibitors and/or statins.

In another embodiment, the present invention provides a method for determining the risk of mortality in a patient with diabetes mellitus, comprising the steps of

a) determining a set of biological parameters obtained from the patient, wherein the set of biological parameters comprises the following variables:

the patient's gender,

the patient's age,

at least one indicator of myocardial performance, wherein the at least one indicator of myocardial performance is defined by the patient's blood concentration of one or more natriuretic peptide(s),

an indicator of renal performance, wherein the indicator of renal performance is defined by the patient's blood concentration of renin,

the duration of diabetes mellitus in the patient defined in years,

an indicator of lipid metabolism, wherein the indicator of lipid metabolism is defined by the patient's blood activity of lipoprotein associated phospholipase A2 (Lp-PLA2), and

an indicator of blood calcium and/or phosphate metabolism, wherein the indicator of blood calcium and/or phosphate metabolism is defined by the patient's blood concentration of vitamin $D_3$;

b) setting the biological parameter into correlation, thereby providing a risk score, wherein the risk score is indicative for all-cause mortality of the patient within the next years, and wherein the risk score is indicative for a medical treatment of the patient to reduce the patient's risk of all-cause mortality,
wherein the risk score is a weighted risk score $Y_2$ and the biological parameter are set into correlation by using the following formula, thereby providing the weighted risk score $Y_2$:

$$Y_2 = 0.56 * \text{variable } 1 + 0.22 * \text{variable } 2 + 1.95 * (\text{variable } 3 - 4.17) + 0.41 * (\text{variable } 4 - 5.97) + 0.26 * (\text{variable } 5 - 3.28) + 0.85 * (\text{variable } 6 - 6.15) - 0.52 * (\text{variable } 7 - 2.60)$$

wherein

variable 1 is defined as equal to 1 if the patient's gender is male and equal to 0 if the patient's gender is female,

variable 2 is defined by the log value of the duration of diabetes mellitus in years,

variable 3 is defined by the log value of the patient's age in years,

variable 4 is defined by the log value of the patient's blood concentration of N-terminal pro-B-type natriuretic peptide (Nt-proBNP) in ng/L,

variable 5 is defined by the log value of the patient's blood concentration of renin in pg/ml,

variable 6 is defined by the log value of the patient's blood activity of lipoprotein associated phospholipase A2 (Lp-PLA2) in U/L,

variable 7 is defined by the log value of the patient's blood concentration of vitamin $D_3$, preferably of the patient's blood concentration of 25(OH) vitamin $D_3$, in ng/L,

wherein all log values are calculated to the basis e, and wherein all numeric values in the formula providing the weighted risk score $Y_2$ are defined with a deviation of 50 %, preferably with a deviation of 20 %, more preferably with a deviation of 10 %, and most preferably with a deviation of 5 %.

Here, the "numeric values in the formula providing the weighted risk score $Y_2$" are to be understood as the following numeric values: 0.56, 0.22, 1.95, 4.17, 0.41, 5.97, 0.26, 3.28, 0.85, 6.15, 0.52 and 2.60.

Equally preferred is that all numeric values in the formula providing the weighted risk score $Y_2$ are defined without any deviation.

Moreover, in the context of the present invention, all log values are calculated to the basis e.

As with the simplified risk score $Y_1$, it has surprisingly been found in the context of the present invention that also the weighted risk score $Y_2$ can be correlated with different classes of the risk of mortality, preferably with a class of low risk of mortality, a class of moderate risk of mortality, and a class of high risk of mortality.

Therefore, in a preferred embodiment, a weighted risk score $Y_2$ in the range of less than or equal to 0 indicates a low risk of mortality, a weighted risk score $Y_2$ in the range of 0 to 1 indicates a moderate risk of mortality, and a weighted risk score $Y_2$ in the range of equal to or more than 1 indicates a high risk or mortality.

In the context of the present invention, it is to be understood that the term "equal to" may comprise a deviation of $\pm$ 0.5, preferably of $\pm$ 0.25, more preferably of $\pm$ 0.1.

Moreover, it is to be understood that those patients with a weighted risk score $Y_2$ in the range of less than or equal to 0 fall into the 1st tertile of the given cohort or population (minimum to 33rd percentile), those patients with a weighted risk score $Y_2$ in the range of 0 to 1 fall into the 2nd tertile of the cohort (33rd percentile to 66th percentile), while those patients with a weighted risk score $Y_2$ in the range of more than or equal to 1 fall into the 3rd tertile of the cohort (66th percentile to maximum).

The term "percentile" generally means the value of a variable below which a certain percent of observations fall. For example, the 33rd percentile is the value below which 33 percent of the observations may be found. In the present invention, the cut off values off the 1st, 2nd and 3rd tertile have been determined as exemplified in the examples of the present invention.

Disclosed is a method for determining the risk of mortality in a patient with diabetes mellitus, comprising the steps of

a) analysing a set of biological parameters obtained from the patient, wherein the set of biological parameters comprises the following variables:

the patient's gender,

the patient's age,

at least one indicator of myocardial performance,

an indicator of renal performance,

the duration of diabetes mellitus in the patient defined in years,

an indicator of lipid metabolism,

and an indicator of blood calcium and/or phosphate metabolism,

b) setting the biological parameter into correlation, thereby providing a risk score, wherein the risk score is indicative for all-cause mortality of the patient within the next years, and wherein the risk score is indicative for a medical treatment of the patient to reduce the patient's risk of all-cause mortality,

wherein the risk score is a weighted risk score $Y_2$ having the definition provided above.

According to a preferred embodiment of the invention, the patient's risk of cardiovascular mortality is caused by a disease of the arterial tree. Preferably, the cardiovascular disease is due to atherosclerosis and/or to thrombosis.

In a further preferred embodiment, the risk score of the present invention is indicative for all-cause mortality within the next 3 years, preferably within the next 5 years, more preferably within the next 10 years.

Even more preferably, the risk score of the present invention is indicative for cardiovascular mortality within the next 3 years, preferably within the next 5 years, more preferably within the next 10 years.

In the context of the present invention, it has surprisingly be found that both the simplified risk score $Y_1$ and the weighted risk score $Y_2$ have excellent discriminatory power for prediction and determining the risk of all-cause mortality and cardiovascular mortality, as exemplified in the Examples section.

The risk score determined by the methods of the present invention allows an appropriate medical treatment of the patient to reduce the patient's risk of all-cause mortality, preferably of cardiovascular mortality. This medical treatment is generally in form of administering drugs.

According to the present invention, the medical treatment of a patient includes the administration of a therapeutically effective dose or pharmaceutically active compound in form of a pharmaceutical composition, e.g. a drug, which prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50 % of the population) and LD50 (the dose lethal to 50 % of the population). The dose ratio between therapeutic and toxic effects is the therapeutic index, and it can be expressed as the ratio, LD50/ED50.

The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.

The term "drug" generally means any composition formulated in solid, liquid (or gaseous) form which can be administered to a patient. Said composition generally comprises a therapeutically active compound optionally together with suitable auxiliary compounds such as diluents or carriers or further ingredients. In this context, it is distinguished between auxiliary compounds, i.e. compounds which do not contribute to the effects elicited by the compound upon application of the composition for its desired purpose, and further ingredients, i.e. compounds which contribute a further effect or modulate the effect of the therapeutic effective ingredient. Suitable diluents and/or carriers depend on the purpose for which the composition is to be used and the other ingredients. The person skilled in the art can determine such suitable diluents and/or carriers without further ado. Examples of suitable carriers and/or diluents are disclosed elsewhere herein. Drugs suitable for oral administration may be presented as discrete units such as capsules, cachets, tablets, or lozenges. Other compositions include suspensions in aqueous liquors or non-aqueous liquids such as an syrup, an elixir, or an emulsion.

As used herein, an effective amount of the administered drug is a dosage large enough to produce the desired therapeutic effect to reduce the risk of all-cause mortality and/or cardiovascular mortality. An effective amount is not, however, a dosage so large as to cause adverse side effects. Generally, an effective amount may vary with the patient's age, condition, weight and sex, as well as the extent of the condition being treated, and can be determined by one of skill in the art. The dosage may be adjusted by the individual practitioner in the event of any complication.

Preferably, in the context of the present invention, the medical treatment of the patient is in form of administering drugs, preferably wherein the drugs are for treating hyperlipidaemia, regulating blood pressure, modifying the heart rate, and/or for providing glycaemic control, and/or for inhibiting blood coagulation in diabetic patients.

The term "hyperlipidaemia" (also referred to as "hyperlipoproteinemia" and/or "hyperlipidaemia") generally means a disease which involves abnormally elevated levels of any or all lipids and/or lipoproteins in the blood. Hyperlipidaemias are divided in primary and secondary subtypes. Primary hyperlipidaemia is usually due to genetic causes (such as a mutation in a receptor protein), while secondary hyperlipidaemia arises due to other underlying causes such as diabetes. Lipid and lipoprotein abnormalities are common in the general population, and are regarded as a modifiable risk factor for cardiovascular disease due to their influence on atherosclerosis.

Preferably, treating hyperlipidaemia according to the present invention means a treatment which results in a reduction of the patient's blood lipids and/or lipoproteins, preferably in reducing the concentration of cholesterol.

The term "glycaemic control" generally refers to any medical treatment that aims at controlling the levels of blood glucose (blood sugar) in a person with diabetes mellitus. Blood glucose levels can be measured by means of a glucose meter, with the result either indicated in mg/dL (milligrams per deciliter in the USA) or mmol/L (millimoles per litre in Canada and Europe) of blood. The average glucose level of a healthy person is around 4.5 to 7.0 mmol/L (80 to 125 mg/dL).

Advantageously, the present invention provides reliable methods for determining the risk of mortality in patients with diabetes mellitus. The identification of high risk patients allows for a closer monitoring of this group so that preventive treatments can be administered to those patients with the greatest need. This suggests specific preventive measures of medial treatment: patients with a determined intermediate (or moderate) or high risk of mortality should receive therapies that aim, for example, at reducing the concentration of cholesterol in the blood, at reducing the patient's blood glucose concentration, or at reducing the patient's blood pressure. Thus, the present invention contributes to the development of individualized treatment regimens.

In a preferred embodiment of the invention, the medical treatment is in form of administering drugs, wherein the drugs are for reducing the patient's blood concentration of cholesterol, for reducing the patient's blood pressure, and/or for reducing the patient's blood glucose concentration.

"Medical treatment" according to the present invention is to be understood as any treatment of secondary prevention which improves the patient's life span and/or health. The medical treatment of the invention is preferably applied to patients for whom a high risk of mortality has been determined by the methods of the present invention or as described herein. This risk of mortality can be a risk of all-cause mortality or, preferably, a risk of cardiovascular mortality.

Accordingly, in a preferred embodiment, the risk score determined by the methods of the present invention or as described herein is indicative for a medical treatment of patients with a high risk of mortality.

Patients identified with a high risk of mortality by the methods of the present invention or as described herein participate in particular from this indication since this group of patients has so far, in most cases, not been subjected to optimal medical treatments for reducing all-cause or, preferably, cardiovascular mortality. Hence, the methods of the present invention are particularly useful for patients with diabetes mellitus which are categorized with a high risk of mortality.

More preferably, the medical treatment and/or the medical treatment of secondary prevention is applied more intensively in patients with a high risk of mortality than in patients with a moderate risk of mortality. Consequently, the medical treatment and/or the treatment of secondary prevention may be the more intense, the higher the patient's risk of mortality has been determined by the methods of the present invention.

In another aspect, the present invention provides a computer program product directly loadable into the internal memory of a digital computer, comprising software code portions for implementing the calculation of the mortality risk score according to the present invention when said product is run on a computer.

According to the invention, a computer program product is provided for performing one of the previously explained methods, when the product is run on a computer. The computer program product is preferred to be directly loadable into the internal memory of a digital computer and comprises software code portions for implementing the calculation of the risk score.

The computer program product may be a computer program preferably stored on a machine readable storage medium like RAM, ROM, or on a removable CD-ROM, flash memory, DVD or USB-stick. The computer program may be provided on a server to be downloaded via for example a data network such as the internet or another transfer system such as a phone line or a wireless transfer connection. Additionally, or alternatively, the computer program product may be a network of computer implemented computer programs such as a client/server system or a cloud computing system, an embedded system with a computer program or an electronic device like a smart phone or a personal computer on which a computer program is stored, loaded, running, exercised, or developed.

In one implementation, the information on the set of biological parameters obtained from the patient is entered into an input device of a computer, an embedded system, an electronic device or a smart phone via a keyboard, a touch screen or a voice interface. In another implementation, the information on the set of biological parameters is measured by adequate measuring devices and transmitted via the internet or another transfer system such as a phone line or a wireless transfer connection to a remote station such as a computer.

In one implementation, the information on the calculated risk score is provided on an output medium, stored in a memory means or transferred to a remote station. In one embodiment, the information on the risk score is displayed on a screen or display as output medium.

Preferably, the computer program product of the present invention comprises software code portions for implementing the calculation of the simplified risk score $Y_1$ and/or the weighted risk score $Y_2$ as defined in the context of the present invention.

In yet another preferred embodiment, the computer program product of the present invention comprises software code portions for implementing the calculation of the weighted risk score $Y_2$ as defined in the context of the present invention.

It is to be understood that the definitions and explanations of the methods, measurements, and terms made above apply mutatis mutandis for all aspects described in this specification in the following except as otherwise indicated.

The following Figures and Examples are intended to illustrate various embodiments of the present invention. As such, the specific modifications discussed therein are not to be understood as limitations of the scope of the invention. It will be apparent to the person skilled in the art that various equivalents, and modifications may be made without departing from the invention, and it is thus to be understood that such equivalent embodiments are to be included herein.

**FIGURES**

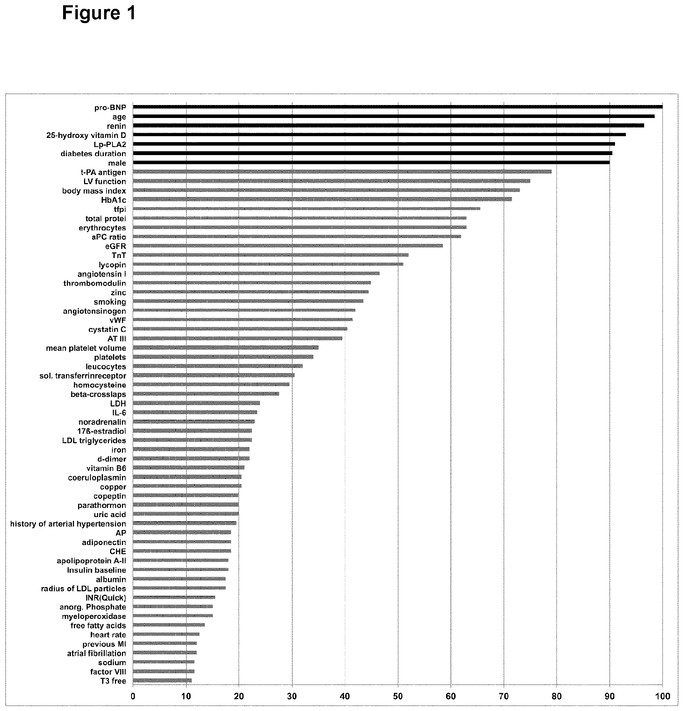

**Figure 1: Variable selection by a bootstrap resampling procedure based on Cox regression analysis** (cut off level for selection: 80%). To avoid collinearity the following variables were selected for the bootstrap procedure due to their highest univariate predictive value (defined by the hazard ratio for 1-SD change derived from Cox regression) from clusters with close correlations (r > 0.4): Cystatin (NOT selected: BUN, creatinine, ADMA, SDMA, eGFR), free fatty acids, LDL triglycerides and Lp-PLA2 (NOT selected: total cholesterol, HDL triglycerides, lipoprotein(a), free glycerol, apolipoprotein A-I, HDL cholesterol ester, HDL phospholipid, HDL cholesterol, cholesterol ester, LDL cholesterol, apolipoprotein E, LDL apolipoprotein B, apolipoprotein B, LDL phospholipid, triglycerides, HDL free cholesterol, apolipoprotein C-III, a-Tocopherol, phospholipid, VLDL apolipoprotein B, VLDL cholesterol ester, VLDL cholesterol, LDL free cholesterol, VLDL free cholesterol, apolipoprotein A-II), body mass index (NOT selected: waist-to-hip ratio), antithrombin III (NOT selected: retinol), lycopin (NOT selected: alpha_carotin, beta-carotin, lutein + zeaxanthin, all-trans beta-carotin, cis beta-carotin, beta_cryptoxanthin), noradrenalin (NOT selected: adrenalin), iron (NOT selected: ferritin, transferrin), erythrocytes (NOT selected: hematocrit, hemoglobin, MCV, MCH, MCHC), factor VII (NOT selected: activated factor VII), LH (NOT selected: FSH), coeruloplasmin, IL-6 (NOT selected: fibrinogen, serum amyloid A, LBP, hsCRP), INR(Quick) (NOT selected: endogenous thrombin potential (ETP), prothrombin fragment 1+2, aPTT), glycosylated hemoglobin (NOT selected: fasting glucose, glucose 1 and 2h post OGTT, fasting insulin, insulin 1 and 2h post OGTT, proinsulin 1 and 2h post OGTT, C-peptide 1 and 2h post OGTT), cholinesterase (NOT selected: gamma GT, ALT, AST, total bilirubin), osteocalcin, beta-crosslaps, t-PA antigen (NOT selected: t-PA activity, PAI-1 activity, PAI-1 antigen), NT-proBNP (NOT selected: homoarginine), renin (NOT selected: aldosterone), free T3 (NOT selected: free T4, TSH), vitamin B6 (NOT selected: folic acid, vitamin B12, vitamin B1), 25-hydroxy vitamin D (NOT selected: 1-25-dihydroxy vitamin), atrial fibrillation, previous myocardial infarction and duration of diabetes; Variables with a high univariate hazard ratio (NT-proBNP, TnT, cystatin C, estimated glomerular filtration rate, copeptin) were included in the bootstrapping resampling procedure despite correlation coefficients > 0.4. In case of close correlations selection of the respective variables were tested with and without the correlating variable. quick prothrombin time; BUN, blood urea nitrogen; HDL, high-density lipoprotein; BMI, body mass index; LDL, low-density lipoprotein; ALT alanin-aminotransferase; hsCRP, high-sensitivity C-reactive protein.

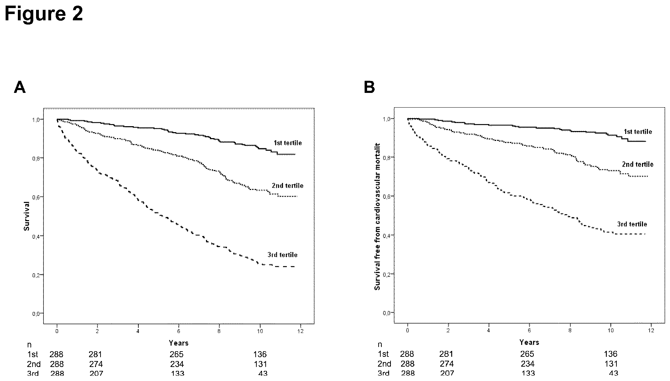

**Figure 2: Kaplan-Meier estimates of mortality.** Kaplan-Meier estimates for tertiles of VILDIA score for all-cause mortality (A) and cardiovascular mortality (B) (all P<0.001 between all tertiles, log rank test).

**Figure 3: Risk prediction of simplified risk score and association with established cardiovascular medication.** The bars show 10-year all-cause (3A) and cardiovascular (3B) mortality stratified by the simplified biomarker score. We further found an inverse association between the simplified risk and aspirin use (for trend, p <0.007, Fig. 3C), beta blocker use (for trend, p < 0.001, Fig. 3D) and statin use (for trend, p < 0.001, Fig. 3E) at discharge. The simplified score was based on the following cut-off values: [male = 1] + [age $\geq$ 75years (86th percentile)=1] + [NT-proBNP $\geq$ 400 ng/L (51th percentile)=1, $\geq$ 2000 ng/L (85th percentile)=2] + [diabetes duration $\geq$ 5 years (81th percentile)=1] + [renin $\geq$ 50 pg/ml (74th percentile)=1)] + [25-OH vitamin $D_3$ $\leq$ 10 ng/L (29th percentile)=1] + [Lp-PLA2 > 450 U/l (43rd percentile)=1].

## EXAMPLES

**Aims.** Cardiovascular risk assessment in patients with diabetes relies on traditional cardiovascular risk factors. However, numerous novel biomarkers have been found to be independent predictors of cardiovascular disease, which might significantly improve risk prediction in diabetic patients. The purpose of this study was to improve prediction of cardiovascular risk in diabetic patients by investigating 135 evolving biomarkers encompassing various pathophysiologic pathways. Based on selected biomarkers a clinically applicable prediction algorithm for long-term cardiovascular mortality in this growing patient population was designed.

**Methods.** We prospectively enrolled 864 diabetic patients of the LUdwigshafen Risk and Cardiovascular health (LURIC) study with a median follow-up of 9.6 years. Independent risk factors were selected using bootstrapping based on a Cox regression analysis.

**Results.** The following seven variables were selected for the final multivariate model: NT-proBNP, age, male sex, renin, diabetes duration, Lp-PLA2 and 25-OH vitamin $D_3$. The risk score based on the aforementioned variables demonstrated an excellent discriminatory power for 10-year survival free from cardiovascular death with a C-statistic of 0.76 (P<0.001), which was significantly better than the established U.K. Prospective Diabetes Study (UKPDS) risk engine (C-statistic=0.64, P<0.001). Net reclassification confirmed a significant improvement of individual risk prediction by 22% (95% confidence interval: 14-30%) compared to the UKPDS risk engine (P<0.001).

**Conclusion.** The VILDIA score based on traditional cardiovascular risk factors and reinforced with novel biomarkers outperforms previous risk algorithms. The new score allows more accurate classification of cardiovascular risk in diabetic patients and an individualized treatment potentially improving survival.

There is broad evidence that diabetes mellitus is associated with elevated all-cause and cardiovascular mortality [1-3]. However, the increase in risk associated with diabetes mellitus varies considerably [4]. Based on novel biomarkers great efforts have been made to improve risk estimation in people with cardiovascular disease [5, 6]. Nevertheless, an up-to-date risk score specifically addressing people with diabetes is not available. Hence, the most widely used algorithm for people with diabetes mellitus is still the U.K. Prospective Diabetes Study (UKPDS) risk score [7]. This algorithm only includes two biomarkers, namely glycated hemoglobin and the total cholesterol to high-density lipoprotein cholesterol ratio [7]. Since the development of the UKPDS score numerous biomarkers have been found to be independent predictors of cardiovascular disease, for example C-reactive protein, Vitamin-D, and renin [8].

Hence, the present analyses aimed to improve the prediction of cardiovascular death in diabetic patients using a variety of evolving biomarkers and to show superior prognostic accuracy compared with the UKPDS risk score and compared with single predictors. We studied participants of the LUdwigshafen Risk and Cardiovascular health (LURIC) study who had a known personal history of diabetes mellitus or were newly diagnosed with diabetes mellitus [9]. LURIC is a large prospective observational study which was designed to investigate clinical and biochemical cardiovascular risk factors [9]. Potential clinical implications of improved risk stratification are a tailored use of preventive strategies with a particular focus on high-risk patients.

## Research Design and Methods

*Study population.* The Ludwigshafen Risk and Cardiovascular Health (LURIC) study comprises 3,316 Caucasian patients referred to the Cardiac Center Ludwigshafen (Germany) for coronary angiography between 1997 and 2000. The detailed study protocol has been previously published [9]. The study protocol complies with the Declaration of Helsinki and was approved by the Ethics Committee of the "Ärztekammer Rheinland-Pfalz". For the present analysis, we chose 864 patients with diabetes. Patients with an acute coronary syndrome at hospital admission were excluded from our analysis. *Clinical definitions.* Diagnosis of diabetes was based on the revised American Diabetes Association criteria [10]. Subjects with increased fasting ($\geq$126 mg/dl) and/or post-challenge (2 h after the 75 g glucose load $\geq$200 mg/dl) glucose or with an HbA1c > 6.5% were diagnosed with diabetes [11]. People with a self-reported or documented history of diabetes or treatment with oral antidiabetics or insulin were considered diabetic (9). Diabetes duration was categorized into newly diagnosed diabetes, duration $\leq$5 years, duration >5 to $\leq$10 years, duration >10 years as previously published [12]. Clinical risk factors such as hypertension, current smoking, lipid disorders and previous myocardial infarction (MI) were diagnosed according to the respective guidelines as previously described [9]. Venous blood samples were drawn under standardized conditions after an overnight fasting period. NT-proBNP was measured by electrochemiluminescence on an Elecsys 2010 (Roche Diagnostics). Plasma renin concentration was determined by an immunoradiometric assay (Active Renin, Nichols Institute Diagnostics, San Juan, Capistrano, CA, USA). Lipoprotein associated phospholipase A2 (Lp-PLA2) activity was measured by use of the Azwell Auto PAF-AH reagent set (Azwell). Serum levels of 25-OH vitamin $D_3$ were measured using a radioimmunoassay (DiaSorin SA, Antony, France). Intra and inter-assay coefficients of variation were <10% for these assays.

*Study endpoints and follow-up.* The primary study endpoints were all-cause mortality and cardiovascular mortality. Information on patient outcome was obtained by screening local registries. No patient was lost to follow-up. Death certificates were obtained in 97% of deceased participants and used to classify them into those who died of cardiovascular versus non-cardiovascular causes as previously described [9]. This classification was performed independently by two experienced clinicians who were blinded to the study participants, except for information that was required to classify the cause of death.

*Statistical analysis.* Discrete data was presented as counts and percentages, continuous data were presented as median and interquartile range. Cox proportional hazard regression analysis was used to evaluate the effect of the investigated variables on survival. Continuous variables were log-transformed before entering analysis. A bootstrap resampling procedure was used to identify best-fitting variables for the final multivariate Cox regression model. Before inclusion of variables into the bootstrapping procedure collinearity between continuous variables was assessed using Pearson correlation coefficients (data not shown). In case of close correlation only the strongest univariate predictor of a cluster (defined by the highest univariate hazard ratio for an increase of 1 standard deviation (SD)) entered the selection procedure. Samples with a size of 100% of the original cohort were chosen for repeats. 100 repeats with backward selection and 100 repeats with forward selection (with a P<0.1 for selection) were executed. Variables selected in more than 80% of all repeats were included in the final multivariable model. Harrell's C-statistic was used to estimate the discriminatory power of the designed risk score. For further analysis the cohort was stratified by tertiles of the biomarker risk score. An improvement of individual risk prediction was examined by the net reclassification improvement (NRI). Kaplan-Meier analysis (log-rank test) was applied to verify the time-dependent discriminative power of risk scores. Cut-off values for a simplified score using categorized variables were estimated using chi-square values derived from Martingale residuals of the Cox model for all possible cut-off values. For optimized clinical use these calculated cut-off values were rounded and extreme values were avoided. Chi square test for linear to linear association was used to assess the trend between medication and simplified risk score categories. Two-sided P-values <0.05 were used to indicate statistical significance. The STATA software package (Stata Statistical Software: Release 11, StataCorp LP, USA) and SPSS 17.0 (IBM SPSS, USA) was used for all analysis.

## Results

*Baseline characteristics and univariate survival analysis.* We prospectively enrolled 864 diabetic patients of the LURIC study into our study. Forty-three percent of all patients (n=369) died during a median follow-up of 9.6 years (IQR: 5.5- 10.4) corresponding to 6837 overall person-years of follow-up. Sixty-eight percent of deaths in patients were due to cardiovascular causes. Thirty-five percent of patients experienced myocardial infarction before study inclusion. Detailed baseline characteristics of the study population are displayed in *supplemental table 1.* In the univariate analysis, the strongest adverse risk factors for long-term outcome in diabetic patients were NT-proBNP with a crude hazard ratio (HR) of 2.04 (95% confidence interval [CI] 1.82- 2.28; P<0.001) per 1-SD increase and age (crude HR 1.51; 95%CI 1.35- 1.70; P<0.001; *table 1*). 25-OH vitamin $D_3$ had a strong inverse effect on mortality with a HR of 0.68 per 1-SD increase (95%CI 0.61- 0.75 P<0.001; *table 1*).

*Bootstrapping results and multivariable survival analysis.* Variables for the multivariate model were selected using a bootstrapping resampling procedure. NT-proBNP, age, male sex, renin, diabetes duration, Lp-PLA2 and 25-OH vitamin $D_3$ were selected as significant mortality predictors for the final multivariate model (*figure 1*). In this multivariable model male sex and NT-pro-BNP were the strongest adverse risk factors and 25-OH vitamin $D_3$ remained the strongest inverse risk factor (*table 1*).

*Risk score design and comparison with established risk scores.* Furthermore, we generated the Vienna Ludwigshafen DIAbetes (VILDIA) risk score, a weighted risk score for long-term survival using the aforementioned variables obtained by the bootstrapping procedure (*see supplemental method 1 for the formula*). The C-statistic of the VILDIA score indicated an excellent discriminatory power for 10-year survival with a value of 0.75 (P<0.001) for all-cause mortality and for 10-year survival free of cardiovascular death with a value of 0.76 (P<0.001). The discriminatory ability of our score was superior to the previously published UKPDS risk engine [7] for 10-year survival free of cardiovascular death (C-statistic 0.64; P<0.001 for comparison). Furthermore, the VILDIA score was significantly superior to the UKPDS risk engine with respect to improved risk classification with a NRI of 21% (95% CI: 12-30%; P<0.001) for all-cause mortality and of 22% (95% CI: 14-30%; P<0.001) for cardiovascular mortality. Kaplan Meier analysis confirmed the high discriminatory value when plotting tertiles of the new risk score for all-cause mortality (*figure 2A*) and cardiovascular mortality (*figure 2B*). The 10-year survival rates in the first, second and third tertile of the new score were 85%, 63%, and 25%, respectively (P<0.001 between all tertiles). With regard to cardiovascular mortality, a comparable trend for the 10-year survival was observed, with survival rates of 91%, 73%, and 41% in the first, second and third tertile of the new risk score (P≤0.001 between all tertiles).

*Simplified score.* Ultimately, we aimed to create a simplified VILDIA score for optimal clinical use. The design of this simplified score was based on the same variables as the weighted risk score. Continuous variables were dichotomized according to optimal cut-off levels, as described in *figure 3*. Two points were assigned to the variable Nt-proBNP with the strongest predictive value in the multivariate model while one point was allocated to the other variables. The simplified VILDIA score still demonstrated a strong discriminatory power with a C-statistic of 0.72 for all-cause mortality and 0.72 for cardiovascular mortality over 10 years. The discriminatory ability of the simplified VILDIA score was still superior to the UKPDS risk engine (P=0.01). All-cause mortality ranged from 5.3% with zero points to 94.9% with six or more points (*figure 3A*). Cardiovascular mortality ranged from 5.3% to 56.4% (*figure 3B*). With regard to the use of pharmacologic treatment reducing cardiovascular mortality we found inverse associations between the simplified VILDIA score and aspirin use (for trend, p <0.007, *figure 3C*), beta blocker use (for trend, p < 0.001, *figure 3D*) and statin use (for trend, p < 0.001, *figure 3E*) at discharge.

## TABLES

**Table 1: Unadjusted and adjusted effects on mortality** - Cox proportional hazard model of variables selected by bootstrapping. Hazard ratios (HR) refer to a 1-SD increase in continuous variables, a reduction in one category of LVEF and a increase in one category of diabetes duration. HRs are adjusted (adj.) for all variables selected by bootstrapping i.e. for male sex, age, pro-BNP, HbA1c, renin, 25-OH vitamin $D_3$, and lipoprotein associated phospholipase A2 (Lp-PLA2). SD - standard deviation;

| Variables | SD | Crude HR (95%CI) | P-value | Adj. HR (95%CI) | P-value |
|---|---|---|---|---|---|
| Nt-proBNP | 2395.24 | 2.04 (1.82- 2.28) | **<0.001** | 1.83 (1.62- 2.06) | **<0.001** |
| Age | 9.12 | 1.51 (1.35- 1.70) | **<0.001** | 1.33 (1.18- 1.50) | **<0.001** |
| Male sex | ---- | 1.48 (1.17- 1.87) | **0.001** | 1.74 (1.37-2.23) | **<0.001** |
| Renin | 244.84 | 1.47 (1.34- 1.62) | **<0.001** | 1.40 (1.27- 1.54) | **<0.001** |
| Diabetes duration | ---- | 1.29 (1.19- 1.41) | **<0.001** | 1.24 (1.14- 1.36) | **<0.001** |
| Lp-PLA2 | 131.86 | 1.20 (1.08- 1.33) | **<0.001** | 1.26 (1.13- 1.40) | **<0.001** |
| 25-OH vitamin $D_3$ | 8.64 | 0.68 (0.61- 0.75) | **<0.001** | 0.75 (0.67- 0.83) | **<0.001** |

**Table 2: Reclassification table -** Reclassification table comparing the VILDIA score with the UKPDS score for A) all-cause and B) cardiovascular mortality

| A | | | | | |
|---|---|---|---|---|---|
| | UKDPS | VILDIA score | | | |
| | Risk class | Low | Moderate | High | Total |
| No event (n=495) | Low | 67% (145) | 26% (56) | 7% (15) | 100% (216) |
| | Moderate | 42% (74) | 45% (78) | 13% (23) | 100% (175) |
| | High | 23% (24) | 43% (45) | 34% (35) | 100% (104) |
| Event (n=369) | Low | 35% (25) | 32% (23) | 33% (24) | 100% (72) |
| | Moderate | 12% (14) | 40% (45) | 48% (54) | 100% (113) |
| | High | 3% (6) | 22% (41) | 75% (137) | 100% (184) |
| B | | | | | |
| | UKDPS | VILDIA score | | | |
| | Risk class | Low | Moderate | High | Total |
| No event (n=619) | Low | 66% (154) | 25% (60) | 9% (21) | 100% (235) |
| | Moderate | 38% (82) | 55% (97) | 17% (38) | 100% (217) |
| | High | 16% (26) | 33% (56) | 51% (85) | 100% (167) |
| Event (n=245) | Low | 30% (16) | 36% (19) | 34% (18) | 100% (53) |
| | Moderate | 8% (6) | 37% (26) | 55% (39) | 100% (71) |
| | High | 3% (4) | 25% (30) | 72% (87) | 100% (121) |
| Data are given as % row (n). | | | | | |

**Conclusions**

We developed the VILDIA score, a multimarker score for patients with diabetes, which provides an excellent discriminatory power for the prediction of 10-year survival with a C-statistic of 0.75. The final set of six variables was selected using a bootstrapping resampling procedure from a large set of 135 clinical and biochemical markers encompassing various pathophysiologic pathways and reflecting different aspects of the vulnerable patient with diabetes. The clinical relevance of this new multimarker score was further emphasized by a significant improvement of net reclassification by 21%, which reflects the proportion of individuals, who may benefit from an improved risk prediction compared to the regularly used UKPDS risk engine. The new VILDIA score predicted long-term mortality irrespective of the presence of a previous MI.

In general, diabetes is associated with an approximately three times higher risk of cardiovascular events [13]. Accordingly, similar preventive measures such as stricter target values for cholesterol or blood pressure have been proposed [14]. However, a risk increase of diabetic patients equivalent to established coronary artery disease has been recently questioned [15]. Therefore, a refinement of cardiovascular risk estimation in patients with diabetes is warranted to manage the cardiovascular risk of patients with diabetes in an individualized form. So far the UKPDS risk engine has been the best available tool to predict the cardiovascular risk of patients with diabetes. This score included clinical characteristics including age, sex, ethnicity, smoking, HbA1c, systolic blood pressure and cholesterol [7]. However, the new VILDIA score outperformed this score by adding a set of selected biomarkers. As shown by a superior predictive value of the score compared to single risk markers this score may also reflect the multifactorial pathogenesis of cardiovascular events more accurately than a single marker. Robustness of the selection of markers (*figure 1*) and independency of their predictive value as shown in the multivariable model (*table 1*) strengthened the overall predictive value of the score. Furthermore, selection from an initial set of more than 100 biomarkers strongly increases the probability of covering the majority of relevant pathogenetic factors. The predictive value of VILDIA was not influenced by previous MI and, even more importantly, the presence of a previous MI did not increase the predictive value of the VILDIA score (data not shown).

All (bio)markers included in the VILDIA score have been shown to be associated with cardiovascular risk. Duration of diabetes is associated with increased risk of major coronary heart disease events [16] and has been repeatedly identified as a potent predictor of all-cause and cardiovascular mortality [12, 17, 18]. ProBNP and its N-terminal fragment Nt-proBNP are one of the strongest and most robust predictors of mortality in the cardiovascular field, particularly in patients with heart failure [19] and acute coronary syndrome [20, 21]. Natriuretic peptides may also be important predictors of cardiovascular and all-cause mortality in patients with diabetes [22-24]. In our diabetes cohort, Nt-proBNP has been the most robust parameter in the bootstrapping resampling procedure, even more robust than left ventricular function measured by echocardiography (*figure 1*). Renin, part of the renin-angiotensin-aldosterone system (RAAS), is not only involved in development of arterial hypertension [25] but also in the progression of cardiovascular diseases and is associated with cardiovascular events [26, 27]. Conflicting data derived from the Framingham Heart Study where renin was associated with mortality but not with incidence of cardiovascular disease [28, 29]. Furthermore, direct inhibition of renin in addition to standard RAAS inhibition in patients with diabetes has not shown to be beneficial with regard to a composite endpoint including cardiovascular and renal events [30]. 1,25-OH vitamin $D_3$, the active form of vitamin D, requires sunlight as well as conversion in the liver and the kidney. 25-OH vitamin $D_3$, the major circulating form, may be preferred as biomarker. Apart from its role in skeletal pathologies vitamin D deficiency also affects extraskeletal diseases including cardiovascular diseases. Particularly, in patients with metabolic syndrome or diabetes, low levels of vitamin D have been associated with increased cardiovascular mortality [31, 32]. Finally, activity of Lp-PLA2, also described as platelet-activating factor acetylhydrolase, is closely correlated with cardiovascular risk factors suggesting an important role in atherogenesis [33]. Experimental data indicated that inhibition of Lp-PLA2 may prevent progression of atherosclerotic plaques [34]. Furthermore, Lp-PLA2 is associated with cardiovascular mortality [35] and predicts cardiovascular outcome in diabetic patients [36].

While scores are superior in risk prediction compared to single risk factors they are underutilized in clinical practice [6, 37]. An easy-to-use form such as the simplified VILDIA score using categorized variables based on optimized cut-offs is therefore of utmost importance for daily clinical use. The simplified VILDIA score shows a comparable predictive accuracy and is able to fulfill the most important aim of ranking patients [38] into low 10-year fatal cardiovascular risk (<10%) with zero or one point, intermediate risk (20-30%) with two to three points and high risk (about 50% or more) with four or more points. The refinement of cardiovascular risk estimation for the individual patients as evidenced by an improved NRI may lead to a more accurate treatment of the individual patient. A negative correlation between the VILDIA score and aspirin, statin and beta-blocker use suggests that there may be potential to optimize the therapy. E.g. less than 30% of patients with a simplified VILDIA score of ≥ 6 received statins. However, this high-risk group has a 10-year cardiovascular mortality of more than 50%. While there is an ongoing discussion about an increased incidence of diabetes in patients on statins they certainly reduce cardiovascular events in patients with known diabetes [39]. Likewise, aspirin and beta-blockers, both reducing cardiovascular risk, have been prescribed with the lowest frequency in patients with a high VILDIA score suggesting a benefit of an individualized risk management based on the VILDIA score. Nevertheless, little consensus exists concerning the question whether diabetic patients without overt cardiovascular disease should receive antiplatelet therapy [40]. Another open question is whether diabetic patients will benefit from screening for silent coronary artery disease [4]. Trying to answer these questions it seems essential to find those patients, who will most likely profit from expensive screening and intervention strategies. The novel VILDIA-score appears to provide this information at affordable costs.

The lack of external validation is a relevant limitation of this study. However, assessment of more than 100 biomarkers in a second cohort is hardly accomplishable. Replication of the score using selected variables only will be required in an external cohort before clinical use as varying population characteristics may lead to different risk estimates. Further, a long-term follow-up implicates that patients have been selected about 10 years ago. Therefore, measures of secondary prevention may be used in a different way nowadays. Another obstacle before clinical application may be significant costs due to measurement of a set of biomarkers. However, the set has been limited to the most important markers and resulted in a superior predictive value. However, cost-effectiveness analysis will be needed before routine use. Strengths of this study are the comprehensive set of biomarkers and a long-term follow-up of 10 years.

In conclusion, the VILDIA score has confirmed the hypothesis that a multi-biomarker score may improve risk estimation in patients with diabetes. Assessment of a set of independent biomarkers may reflect the multi-factorial pathogenesis of cardiovascular events better than single risk factors and scores based on routine variables. A more individualized treatment based on the VILDIA score will provide the potential benefit of improving the therapy of high-risk patients as well as avoiding overtreatment in low-risk patients.

**References**

[1] Emerging Risk Factors C, Seshasai SR, Kaptoge S, et al. (2011) Diabetes mellitus, fasting glucose, and risk of cause-specific death. N Engl J Med 364: 829-841.

[2] Schramm TK, Gislason GH, Kober L, et al. (2008) Diabetes patients requiring glucose-lowering therapy and nondiabetics with a prior myocardial infarction carry the same cardiovascular risk: a population study of 3.3 million people. Circulation 117: 1945-1954.

[3] Haffner SM, Lehto S, Ronnemaa T, Pyorala K, Laakso M (1998) Mortality from coronary heart disease in subjects with type 2 diabetes and in nondiabetic subjects with and without prior myocardial infarction. N Engl J Med 339: 229-234.

[4] Bloomgarden ZT (2011) Diabetes and cardiovascular disease. Diabetes Care 34: e24-30.

[5] Morrow DA (2010) Cardiovascular risk prediction in patients with stable and unstable coronary heart disease. Circulation 121: 2681-2691.

[6] Goliasch G, Kleber ME, Richter B, et al. (2012) Routinely available biomarkers improve prediction of long-term mortality in stable coronary artery disease: the Vienna and Ludwigshafen Coronary Artery Disease (VILCAD) risk score. Eur Heart J 33: 2282-2289.

[7] Stevens RJ, Kothari V, Adler AI, Stratton IM, United Kingdom Prospective Diabetes Study G (2001) The UKPDS risk engine: a model for the risk of coronary heart disease in Type II diabetes (UKPDS 56). Clin Sci (Lond) 101: 671-679.

[8] Roos CJ, Quax PH, Jukema JW (2012) Cardiovascular metabolic syndrome: mediators involved in the pathophysiology from obesity to coronary heart disease. Biomark Med 6: 35-52.

[9] Winkelmann BR, Marz W, Boehm BO, et al. (2001) Rationale and design of the LURIC study--a resource for functional genomics, pharmacogenomics and long-term prognosis of cardiovascular disease. Pharmacogenomics 2: S1-73.

[10] Sacks DB, Arnold M, Bakris GL, et al. (2011) Guidelines and recommendations for laboratory analysis in the diagnosis and management of diabetes mellitus. Diabetes Care 34: e61-99.

[11] Silbernagel G, Sourij H, Grammer TB, et al. (2012) Isolated post-challenge hyperglycaemia predicts increased cardiovascular mortality. Atherosclerosis 225: 194-199.

[12] Silbernagel G, Rosinger S, Grammer TB, et al. (2012) Duration of type 2 diabetes strongly predicts all-cause and cardiovascular mortality in people referred for coronary angiography. Atherosclerosis 221: 551-557.

[13] Stamler J, Vaccaro O, Neaton JD, Wentworth D (1993) Diabetes, other risk factors, and 12-yr cardiovascular mortality for men screened in the Multiple Risk Factor Intervention Trial. Diabetes Care 16: 434-444.

[14] Cho E, Rimm EB, Stampfer MJ, Willett WC, Hu FB (2002) The impact of diabetes mellitus and prior myocardial infarction on mortality from all causes and from coronary heart disease in men. J Am Coll Cardiol 40: 954-960.

[15] Bulugahapitiya U, Siyambalapitiya S, Sithole J, Idris I (2009) Is diabetes a coronary risk equivalent? Systematic review and meta-analysis. Diabet Med 26: 142-148.

[16] Wannamethee SG, Shaper AG, Whincup PH, Lennon L, Sattar N (2011) Impact of diabetes on cardiovascular disease risk and all-cause mortality in older men: influence of age at onset, diabetes duration, and established and novel risk factors. Arch Intern Med 171: 404-410.

[17] Brun E, Nelson RG, Bennett PH, et al. (2000) Diabetes duration and cause-specific mortality in the Verona Diabetes Study. Diabetes Care 23: 1119-1123.

[18] Nelson RG, Sievers ML, Knowler WC, et al. (1990) Low incidence of fatal coronary heart disease in Pima Indians despite high prevalence of non-insulin-dependent diabetes. Circulation 81: 987-995.

[19] Doust JA, Glasziou PP, Pietrzak E, Dobson AJ (2004) A systematic review of the diagnostic accuracy of natriuretic peptides for heart failure. Arch Intern Med 164: 1978-1984.

[20] Omland T, Persson A, Ng L, et al. (2002) N-terminal pro-B-type natriuretic peptide and long-term mortality in acute coronary syndromes. Circulation 106: 2913-2918.

[21] de Lemos JA, Morrow DA, Bentley JH, et al. (2001) The prognostic value of B-type natriuretic peptide in patients with acute coronary syndromes. N Engl J Med 345: 1014-1021.

[22] van Hateren KJ, Landman GW, Kleefstra N, et al. (2012) The Midregional Fragment of Pro-A-Type Natriuretic Peptide, Blood Pressure, and Mortality in a Prospective Cohort Study of Patients With Type 2 Diabetes (ZODIAC-25). Diabetes Care

[23] Bhalla MA, Chiang A, Epshteyn VA, et al. (2004) Prognostic role of B-type natriuretic peptide levels in patients with type 2 diabetes mellitus. J Am Coll Cardiol 44: 1047-1052.

[24] Tarnow L, Gall MA, Hansen BV, Hovind P, Parving HH (2006) Plasma N-terminal pro-B-type natriuretic peptide and mortality in type 2 diabetes. Diabetologia 49: 2256-2262.

[25] Atlas SA (2007) The renin-angiotensin aldosterone system: pathophysiological role and pharmacologic inhibition. Journal of managed care pharmacy : JMCP 13: 9-20.

[26] Tomaschitz A, Pilz S, Ritz E, et al. (2011) Associations of plasma renin with 10-year cardiovascular mortality, sudden cardiac death, and death due to heart failure. Eur Heart J 32: 2642-2649.

[27] Verma S, Gupta M, Holmes DT, et al. (2011) Plasma renin activity predicts cardiovascular mortality in the Heart Outcomes Prevention Evaluation (HOPE) study. Eur Heart J 32: 2135-2142.

[28] Parikh NI, Gona P, Larson MG, et al. (2007) Plasma renin and risk of cardiovascular disease and mortality: the Framingham Heart Study. Eur Heart J 28: 2644-2652.

[29] Wang TJ, Gona P, Larson MG, et al. (2006) Multiple biomarkers for the prediction of first major cardiovascular events and death. N Engl J Med 355: 2631-2639.

[30] Parving HH, Brenner BM, McMurray JJ, et al. (2012) Cardiorenal end points in a trial of aliskiren for type 2 diabetes. N Engl J Med 367: 2204-2213.

[31] Joergensen C, Reinhard H, Schmedes A, et al. (2012) Vitamin D levels and asymptomatic coronary artery disease in type 2 diabetic patients with elevated urinary albumin excretion rate. Diabetes Care 35: 168-172.

[32] Thomas GN, o Hartaigh B, Bosch JA, et al. (2012) Vitamin D levels predict all-cause and cardiovascular disease mortality in subjects with the metabolic syndrome: the Ludwigshafen Risk and Cardiovascular Health (LURIC) Study. Diabetes Care 35: 1158-1164.

[33] Winkler K, Winkelmann BR, Scharnagl H, et al. (2005) Platelet-activating factor acetylhydrolase activity indicates angiographic coronary artery disease independently of systemic inflammation and other risk factors: the Ludwigshafen Risk and Cardiovascular Health Study. Circulation 111: 980-987.

[34] Wilensky RL, Shi Y, Mohler ER, 3rd, et al. (2008) Inhibition of lipoprotein-associated phospholipase A2 reduces complex coronary atherosclerotic plaque development. Nat Med 14: 1059-1066.

[35] Lp PLASC, Thompson A, Gao P, et al. (2010) Lipoprotein-associated phospholipase A(2) and risk of coronary disease, stroke, and mortality: collaborative analysis of 32 prospective studies. Lancet 375: 1536-1544.

[36] Hatoum IJ, Hu FB, Nelson JJ, Rimm EB (2010) Lipoprotein-associated phospholipase A2 activity and incident coronary heart disease among men and women with type 2 diabetes. Diabetes 59: 1239-1243.

[37] Wang TJ (2010) Multiple biomarkers for predicting cardiovascular events: lessons learned. J Am Coll Cardiol 55: 2092-2095.

[38] Chamnan P, Simmons RK, Sharp SJ, Griffin SJ, Wareham NJ (2009) Cardiovascular risk assessment scores for people with diabetes: a systematic review. Diabetologia 52: 2001-2014.

[39] Wang KL, Liu CJ, Chao TF, et al. (2012) Statins, risk of diabetes, and implications on outcomes in the general population. J Am Coll Cardiol 60: 1231-1238.

[40] Nicolucci A, Standl E (2011) Antiplatelet therapy for every diabetic person? Diabetes Care 34 Suppl 2: S150-154.

**Claims**

1. A method for determining the risk of mortality in a patient with diabetes mellitus, comprising the steps of

> a) determining a set of biological parameters obtained from the patient,
> wherein the set of biological parameters comprises the following variables:

>> the patient's gender,
>> the patient's age,
>> at least one indicator of myocardial performance, wherein the at least one indicator of myocardial performance is defined by the patient's blood concentration of one or more natriuretic peptide(s) selected from atrial natriuretic peptide (ANP) and brain natriuretic peptide (BNP) such as pre-proANP, proANP, N-terminal pro-ANP (Nt-proANP), mid-regional-pro-atrial natriuretic peptide (MR-proANP), ANP, pre-proBNP, proBNP, N-terminal pro-B-type natriuretic peptide (Nt-proBNP), or BNP,

an indicator of renal performance, wherein the indicator of renal performance is defined by the patient's blood concentration of renin,

the duration of diabetes mellitus in the patient defined in years,

an indicator of lipid metabolism, wherein the indicator of lipid metabolism is defined by the patient's blood activity of lipoprotein associated phospholipase A2 (Lp-PLA2), and

an indicator of blood calcium and/or phosphate metabolism, wherein the indicator of blood calcium and/or phosphate metabolism is defined by the patient's blood concentration of vitamin $D_3$;

b) setting the biological parameter into correlation, thereby providing a risk score, wherein the risk score is indicative for all-cause mortality of the patient within the next years, and wherein the risk score is indicative for a medical treatment of the patient to reduce the patient's risk of all-cause mortality,

further **characterized in that** the risk score is a simplified risk score $Y_1$, and **in that** the biological parameter are set into correlation by a sum of seven quantities, the sum thereby providing the simplified risk score $Y_1$,

wherein a first quantity is equal to 1 if the patient's gender is male and otherwise equal to 0,

wherein a second quantity is equal to 1 if the patient's age is equal to or more than 75 years and otherwise equal to 0,

wherein a third quantity is equal to 1 if the concentration of the at least one natriuretic peptide is equal to or more than 400 ng/L, equal to 2 if the concentration of the at least one natriuretic peptide is equal to or more than 2000 ng/L and otherwise equal to 0,

wherein a fourth quantity is equal to 1 if the duration of diabetes mellitus is equal to or more than 5 years and otherwise equal to 0,

wherein a fifth quantity is equal to 1 if the concentration of renin is equal to or more than 50 pg/mL and otherwise equal to 0,

wherein a sixth quantity is equal to 1 if the activity of lipoprotein associated phospholipase A2 (Lp-PLA2) is higher than 450 U/L and otherwise equal to 0,

wherein a seventh quantity is equal to 1 if the concentration of vitamin $D_3$ is equal to or less than 10 ng/L and otherwise equal to 0.

2. The method of claim 1, wherein the risk score is indicative for cardiovascular mortality.

3. The method of claim 1 or 2, wherein

   i) the at least one indicator of myocardial performance is defined by the patient's blood concentration of N-terminal pro-B-type natriuretic peptide (Nt-proBNP) or BNP, and
   ii) the indicator of blood calcium and/or phosphate metabolism is defined by the patient's blood concentration of 25(OH)-vitamin $D_3$.

4. The method of claim 1 to 3, wherein a simplified risk score $Y_1$ of
   equal to 0 or equal to 1 indicates a low risk of mortality,
   equal to 2 or equal to 3 indicates a moderate risk of mortality,
   equal to or more than 4 indicates a high risk of mortality.

5. The method of claim 1 to 4, wherein the simplified risk score $Y_1$ is inversely associated with the use of acetylsalicylic acid, beta blocker(s) and/or inhibitors of cholesterol synthesis, preferably CSE inhibitors and/or statins.

6. A method for determining the risk of mortality in a patient with diabetes mellitus, comprising the steps of

   a) determining a set of biological parameters obtained from the patient, wherein the set of biological parameters comprises the following variables:

   the patient's gender,
   the patient's age,
   at least one indicator of myocardial performance, wherein the at least one indicator of myocardial performance is defined by the patient's blood concentration of one or more natriuretic peptide(s) selected from atrial natriuretic peptide (ANP) and brain natriuretic peptide (BNP) such as pre-proANP, proANP, N-terminal pro-ANP (Nt-proANP), mid-regional-pro-atrial natriuretic peptide (MR-proANP), ANP, pre-proBNP, proBNP, N-terminal pro-B-type natriuretic peptide (Nt-proBNP), or BNP,
an indicator of renal performance, wherein the indicator of renal performance is defined by the patient's blood concentration of renin,
the duration of diabetes mellitus in the patient defined in years,
an indicator of lipid metabolism, wherein the indicator of lipid metabolism is defined by the patient's blood activity of lipoprotein associated phospholipase A2 (Lp-PLA2), and
an indicator of blood calcium and/or phosphate metabolism, wherein the indicator of blood calcium and/or phosphate metabolism is defined by the patient's blood concentration of vitamin $D_3$;

b) setting the biological parameter into correlation, thereby providing a risk score, wherein the risk score is indicative for all-cause mortality of the patient within the next years, and wherein the risk score is indicative for a medical treatment of the patient to reduce the patient's risk of all-cause mortality,
further **characterized in that** the risk score is a weighted risk score $Y_2$ and the biological parameters are set into correlation by using the following formula, thereby providing the weighted risk score $Y_2$:

$$Y_2 = 0.56 * \text{variable } 1 + 0.22 * \text{variable } 2 + 1.95 * (\text{variable } 3 - 4.17) + 0.41$$
$$* (\text{variable } 4 - 5.97) + 0.26 * (\text{variable } 5 - 3.28) + 0.85 * (\text{variable } 6 - 6.15)$$
$$- 0.52 * (\text{variable } 7 - 2.60)$$

wherein

a) variable 1 is defined as equal to 1 if the patient's gender is male and as equal to 0 if the patient's gender is female,
b) variable 2 is defined by the log value of the duration of diabetes mellitus in years,
c) variable 3 is defined by the log value of the patient's age in years,
d) variable 4 is defined by the log value of the patient's blood concentration of N-terminal pro-B-type natriuretic peptide (Nt-proBNP) in ng/L,
e) variable 5 is defined by the log value of the patient's blood concentration of renin in pg/ml,
f) variable 6 is defined by the log value of the patient's blood activity of lipoprotein associated phospholipase A2 (Lp-PLA2) in U/L, and
g) variable 7 is defined by the log value of the patient's blood concentration of vitamin $D_3$, preferably of the patient's blood concentration of 25(OH)-vitamin $D_3$, in ng/L,

wherein all log values are calculated to the basis e, and wherein all numeric values in the formula providing the weighted risk score $Y_2$ are defined with a deviation of 50 %, preferably with a deviation of 20 %, more preferably with a deviation of 10 %, and most preferably with a deviation of 5 %.

7. The method of claim 6, wherein a weighted risk score $Y_2$
in the range of less than or equal to 0 indicates a low risk of mortality,
in the range of 0 to 1 indicates a moderate risk of mortality, and
in the range of more than or equal to 1 indicates a high risk or mortality.

8. The method of claims 2 to 7, wherein the patient's risk of cardiovascular mortality is caused by a disease of the arterial tree, preferably by atherosclerosis and/or thrombosis.

9. The method of any of the preceding claims, wherein the risk score is indicative for all-cause mortality, preferably for cardiovascular mortality, within the next 3 years, preferably within the next 5 years, more preferably within the next 10 years.

10. The method of any of the preceding claims, wherein the medical treatment of the patient is in form of administering drugs, preferably wherein the drugs are for treating hyperlipidaemia, regulating blood pressure, modifying the heart rate, and/or for providing glycaemic control, and/or for inhibiting blood coagulation in diabetic patients.

11. The method of claim 10, wherein the drugs are for reducing the patient's blood concentration of cholesterol, for reducing the patient's blood pressure, and/or for reducing the patient's blood glucose concentration.

12. The method of any of the preceding claims, wherein the risk score is indicative for a medical treatment of patients with a high risk of mortality.

13. A computer program product directly loadable into the internal memory of a digital computer, comprising software code portions for implementing the calculation of the mortality risk score according to any one of the preceding claims when said product is run on a computer.

14. The computer program product of claim 13, comprising software code portions for implementing the calculation of the simplified risk score $Y_1$ as defined in claim 1, and/or of the weighted risk score $Y_2$ as defined in claim 6.

**Patentansprüche**

1. Verfahren zum Bestimmen des Sterberisikos in einem Patienten mit Diabetes mellitus, das die Schritte umfasst

a) Bestimmen eines Satzes biologischer Parameter, die von dem Patienten erhalten wurden, wobei der Satz biologischer Parameter die folgenden Variablen umfasst:

das Geschlecht des Patienten,
das Alter des Patienten,
mindestens einen Indikator von myokardialer Leistungsfähigkeit, wobei der mindestens eine Indikator von myokardialer Leistungsfähigkeit durch die Blutkonzentration eines oder mehrerer natriuretischer Peptids/e des Patienten definiert ist, die aus atrialem natriuretischem Peptid (ANP) und hirnnatriuretischem Peptid (BNP) wie z.B. prä-proANP, proANP, N-terminalem pro-ANP (Nt-proANP), mittel-regionalem-pro-atrialem natriuretischem Peptid (MR-proANP), ANP, prä-proBNP, proBNP, N-terminalem pro-B-Typ-natriuretischem Peptid (Nt-proBNP) oder BNP ausgewählt sind,
einen Indikator von Nierenleistungsfähigkeit, wobei der Indikator von Nierenleistungsfähigkeit durch die Blut-Reninkonzentration des Patienten definiert ist,
die Dauer von Diabetes mellitus in dem Patienten, definiert in Jahren,
einen Indikator von Lipidstoffwechsel, wobei der Indikator von Lipidstoffwechsel durch die Blutaktivität von Lipoprotein-assoziierter Phospholipase A2 (Lp-PLA2) des Patienten definiert ist, und
einen Indikator von Blut-Kalzium- und/oder -Phosphatstoffwechsel, wobei der Indikator von Blut-Kalzium- und/oder -Phosphatstoffwechsel durch die Blutkonzentration von Vitamin $D_3$ des Patienten definiert ist;

b) Setzen des biologischen Parameters in Korrelation, wodurch ein Risikoscore bereitgestellt wird, wobei der Risikoscore indikativ für die Gesamtmortalität des Patienten innerhalb der nächsten Jahre ist und wobei der Risikoscore indikativ für eine medizinische Behandlung des Patienten ist, um das Risiko des Patienten für die Gesamtmortalität zu reduzieren,
weiter **dadurch gekennzeichnet, dass** der Risikoscore ein vereinfachter Risikoscore $Y_1$ ist und dass die biologischen Parameter durch eine Summe von sieben Größen in Korrelation gesetzt werden, wobei die Summe dadurch den vereinfachten Risikoscore $Y_1$ bereitstellt,
wobei eine erste Größe gleich 1 ist, wenn das Geschlecht des Patienten männlich ist, und ansonsten gleich 0,
wobei eine zweite Größe gleich 1 ist, wenn das Alter des Patienten gleich oder mehr als 75 Jahre beträgt, und ansonsten gleich 0,
wobei eine dritte Größe gleich 1 ist, wenn die Konzentration des mindestens einen natriuretischen Peptids gleich oder mehr als 400 ng/L ist, gleich 2, wenn die Konzentration des mindestens einen natriuretischen Peptids gleich oder mehr als 2000 ng/L ist, und ansonsten gleich 0,
wobei eine vierte Größe gleich 1 ist, wenn die Dauer von Diabetes mellitus gleich oder mehr als 5 Jahre ist, und ansonsten gleich 0,
wobei eine fünfte Größe gleich 1 ist, wenn die Konzentration von Renin gleich oder mehr als 50 pg/mL ist, und ansonsten gleich 0,
wobei eine sechste Größe gleich 1 ist, wenn die Aktivität der Lipoprotein-assoziierten Phospholipase A2 (Lp-PLA2) höher als 450 U/L ist, und ansonsten gleich 0,
wobei eine siebte Größe gleich 1 ist, wenn die Konzentration von Vitamin $D_3$ gleich oder weniger als 10 ng/L ist, und ansonsten gleich 0.

2. Verfahren nach Anspruch 1, wobei der Risikoscore indikativ für kardiovaskuläre Mortalität ist.

3. Verfahren nach Anspruch 1 oder 2, wobei

i) der mindestens eine Indikator von myokardialer Leistungsfähigkeit durch die Blutkonzentration von N-terminalem pro-B-Typ natriuretischem Peptid (Nt-proBNP) oder BNP des Patienten definiert ist und
ii) der Indikator von Blut-Kalzium- und/oder -Phosphatstoffwechsel durch die Blutkonzentration von 25(OH)-Vitamin $D_3$ des Patienten definiert ist.

4. Verfahren nach Anspruch 1 bis 3, wobei ein vereinfachter Risikoscore $Y_1$ von
gleich 0 oder gleich 1 ein geringes Mortalitätsrisiko anzeigt,
gleich 2 oder gleich 3 ein moderates Mortalitätsrisiko anzeigt,
gleich oder größer als 4 ein hohes Mortalitätsrisiko anzeigt.

5. Verfahren nach Anspruch 1 bis 4, wobei der vereinfachte Risikoscore $Y_1$ umgekehrt mit der Verwendung von Acetylsalicylsäure, Betablocker(n) und/oder Inhibitoren der Cholesterinsynthese, vorzugsweise CSE-Inhibitoren und/oder Statinen, assoziiert ist.

6. Verfahren zum Bestimmen des Mortalitätsrisikos in einem Patienten mit Diabetes mellitus, das die Schritte umfasst

a) Bestimmen eines Satzes biologischer Parameter, die vom Patienten erhalten wurden, wobei der Satz biologischer Parameter die folgenden Variablen umfasst:

das Geschlecht des Patienten,
das Alter des Patienten,
mindestens einen Indikator von myokardialer Leistungsfähigkeit, wobei der mindestens eine Indikator von myokardialer Leistungsfähigkeit durch die Blutkonzentration eines oder mehrerer natriuretischer Peptide des Patienten definiert ist, die aus atrialem natriuretischem Peptid (ANP) und hirnnatriuretischem Peptid (BNP) wie prä-proANP, proANP, N-terminalem pro-ANP (Nt-proANP), mittel-regionalem-pro-atrialem natriuretischem Peptid (MR-proANP), ANP, prä-proBNP, proBNP, N-terminalem pro-B-Typ-natriuretischem Peptid (Nt-proBNP) oder BNP ausgewählt sind,
einen Indikator von Nierenleistungsfähigkeit, wobei der Indikator von Nierenleistungsfähigkeit durch die Blut-Reninkonzentration des Patienten definiert ist,
die Dauer von Diabetes mellitus in dem Patienten, definiert in Jahren,
einen Indikator von Lipidstoffwechsel, wobei der Indikator von Lipidstoffwechsel durch die Blutaktivität von Lipoprotein-assoziierter Phospholipase A2 (Lp-PLA2) des Patienten definiert ist, und einen Indikator von Blut-Kalzium- und/oder -Phosphatstoffwechsel, wobei der Indikator von Blut-Kalzium- und/oder -Phosphatstoffwechsel durch die Blutkonzentration von Vitamin $D_3$ des Patienten definiert ist;

b) Setzen des biologischen Parameters in Korrelation, wodurch ein Risikoscore bereitgestellt wird, wobei der Risikoscore indikativ für die Gesamtmortalität des Patienten innerhalb der nächsten Jahre ist und wobei der Risikoscore indikativ für eine medizinische Behandlung des Patienten ist, um das Risiko des Patienten für die Gesamtmortalität zu reduzieren,
weiter **dadurch gekennzeichnet, dass** der Risikoscore ein gewichteter Risikoscore $Y_2$ ist und die biologischen Parameter durch Verwenden der folgenden Formel in Korrelation gesetzt werden, wodurch der gewichtete Risikoscore $Y_2$ bereitgestellt wird:

$$Y_2 = 0{,}56 * \text{Variable 1} + 0{,}22 * \text{Variable 2} + 1{,}95 * (\text{Variable 3} - 4{,}17) + 0{,}41 * (\text{Variable 4} - 5{,}97) + 0{,}26 * (\text{Variable 5} - 3{,}28) + 0{,}85 * (\text{Variable 6} - 6{,}15) - 0{,}52 * (\text{Variable 7} - 2{,}60)$$

wobei

a) Variable 1 definiert ist als gleich 1, wenn das Geschlecht des Patienten männlich ist, und als gleich 0, wenn das Geschlecht des Patienten weiblich ist,
b) Variable 2 durch den log-Wert der Dauer von Diabetes mellitus in Jahren definiert ist,

c) Variable 3 durch den log-Wert des Alters des Patienten in Jahren definiert ist,

d) Variable 4 durch den log-Wert der Blutkonzentration von N-terminalem pro-B-Typ-natriuretischem Peptid (Nt-proBNP) des Patienten in ng/L definiert ist,

e) Variable 5 durch den log-Wert der Blut-Reninkonzentration des Patienten in pg/ml definiert ist,

f) Variable 6 durch den log-Wert der Blutaktivität von Lipoprotein-assoziierter Phospholipase A2 (Lp-PLA2) des Patienten in U/L definiert ist, und

g) Variable 7 durch den log-Wert der Blutkonzentration von Vitamin $D_3$ des Patienten, vorzugsweise der Blutkonzentration von 25(OH)-Vitamin $D_3$ des Patienten, in ng/L definiert ist,

wobei alle log-Werte zur Basis e berechnet werden und wobei alle numerischen Werte in der Formel, die den gewichteten Risikoscore $Y_2$ bereitstellt, mit einer Abweichung von 50 %, vorzugsweise mit einer Abweichung von 20 %, stärker bevorzugt mit einer Abweichung von 10 % und am meisten bevorzugt mit einer Abweichung von 5 % definiert sind.

**7.** Verfahren nach Anspruch 6, wobei ein gewichteter Risikowert $Y_2$
in dem Bereich von weniger als oder gleich 0 ein geringes Mortalitätsrisiko anzeigt,
in dem Bereich von 0 bis 1 ein moderates Mortalitätsrisiko anzeigt, und
in dem Bereich von mehr als oder gleich 1 ein hohes Risiko oder eine hohe Mortalität anzeigt.

**8.** Verfahren nach den Ansprüchen 2 bis 7, wobei das Risiko des Patienten für kardiovaskuläre Mortalität durch eine Erkrankung des arteriellen Baums, vorzugsweise durch Atherosklerose und/oder Thrombose, verursacht wird.

**9.** Verfahren nach beliebigen der vorhergehenden Ansprüche, wobei der Risikoscore für die Gesamtmortalität, vorzugsweise für kardiovaskuläre Mortalität, innerhalb der nächsten 3 Jahre, vorzugsweise innerhalb der nächsten 5 Jahre, stärker bevorzugt innerhalb der nächsten 10 Jahre, indikativ ist.

**10.** Verfahren nach beliebigen der vorhergehenden Ansprüche, wobei die medizinische Behandlung des Patienten in Form des Verabreichens von Medikamenten ist, vorzugsweise wobei die Medikamente zum Behandeln von Hyperlipidämie, zum Regulieren von Blutdruck, zum Modifizieren der Herzfrequenz und/oder zum Bereitstellen von Glykämiekontrolle und/oder zum Hemmen von Blutgerinnung in Diabetes-Patienten sind.

**11.** Verfahren nach Anspruch 10, wobei die Medikamente zum Reduzieren der Blut-Cholesterinkonzentration des Patienten, zum Reduzieren des Blutdrucks des Patienten und/oder zum Reduzieren der Blut-Glukosekonzentration des Patienten sind.

**12.** Verfahren nach beliebigen der vorhergehenden Ansprüche, wobei der Risikoscore indikativ ist für eine medizinische Behandlung von Patienten mit einem hohen Mortalitätsrisiko.

**13.** Computerprogrammprodukt, das direkt in den internen Speicher eines digitalen Computers geladen werden kann und Softwarecodeteile zum Implementieren der Berechnung des Mortalitätsrisikoscores gemäß einem beliebigen der vorhergehenden Ansprüche umfasst, wenn das Produkt auf einem Computer ausgeführt wird.

**14.** Computerprogrammprodukt nach Anspruch 13, das Softwarecodeteile zum Implementieren der Berechnung des vereinfachten Risikoscores $Y_1$, wie in Anspruch 1 definiert, und/oder des gewichteten Risikoscores $Y_2$, wie in Anspruch 6 definiert, umfasst.


**Revendications**

**1.** Procédé pour déterminer le risque de mortalité d'un patient souffrant de diabète sucré, comprenant les étapes consistant à

a) déterminer un ensemble de paramètres biologiques obtenus chez le patient, où l'ensemble de paramètres biologiques comprend les variables suivantes :

le sexe du patient,
l'âge du patient,
au moins un indicateur de la performance myocardique, où l'au moins un indicateur de la performance myocardique est défini par la concentration d'un ou plusieurs peptides natriurétiques sélectionnés parmi le peptide natriurétique auriculaire (ANP) et le peptide natriurétique cérébral (BNP) tel que le préproANP, le proANP, la partie N-terminale du pro-ANP (Nt-proANP), le peptide mi-régional pronatriurétique auriculaire (MR-proANP), l'ANP, le préproBNP, le proBNP, la partie N-terminale du propeptide natriurétique de type B (Nt-proBNP), ou le BNP, dans le sang du patient

un indicateur de la performance rénale, où l'indicateur de la performance rénale est défini par la concentration de rénine dans le sang du patient,

la durée du diabète sucré chez le patient définie en années,

un indicateur du métabolisme des lipides, où l'indicateur du métabolisme des lipides est défini par l'activité de la phospholipase A2 associée aux lipoprotéines (Lp-PLA2) dans le sang du patient, et

un indicateur du métabolisme du calcium et/ou du phosphate sanguin, où l'indicateur du métabolisme du calcium et/ou du phosphate sanguin est défini par la concentration de vitamine $D_3$ dans le sang du patient ;

b) mettre le paramètre biologique en corrélation, en obtenant ainsi un score de risque, où le score de risque est indicatif de la mortalité toutes causes confondues du patient au cours des prochaines années, et où le score de risque est indicatif d'un traitement médical du patient afin de réduire le risque de mortalité toutes causes confondues du patient,

en outre **caractérisé en ce que** le score de risque est un score de risque simplifié $Y_1$, et **en ce que** les paramètres biologiques sont mis en corrélation par une somme de sept quantités, la somme permettant ainsi d'obtenir le score de risque simplifié $Y_1$,

où une première quantité est égale à 1 si le sexe du patient est masculin et sinon est égale à 0,

où une deuxième quantité est égale à 1 si l'âge du patient est supérieur ou égal à 75 ans et sinon est égale à 0,

où une troisième quantité est égale à 1 si la concentration de l'au moins un peptide natriurétique est supérieure ou égale à 400 ng/l, égale à 2 si la concentration de l'au moins un peptide natriurétique est supérieure ou égale à 2 000 ng/l et sinon est égale à 0,

où une quatrième quantité est égale à 1 si la durée du diabète sucré est supérieure ou égale à 5 ans et sinon est égale à 0,

où une cinquième quantité est égale à 1 si la concentration de rénine est supérieure ou égale à 50 pg/ml et sinon est égale à 0,

où une sixième quantité est égale à 1 si l'activité de la phospholipase A2 associée aux lipoprotéines (Lp-PLA2) est supérieure à 450 U/l et sinon est égale à 0,

où une septième quantité est égale à 1 si la concentration de vitamine $D_3$ est inférieure ou égale à 10 ng/l et sinon est égale à 0.

2. Procédé selon la revendication 1, dans lequel le score de risque est indicatif de la mortalité cardio-vasculaire.

3. Procédé selon la revendication 1 ou 2, dans lequel

i) l'au moins un indicateur de la performance myocardique est défini par la concentration de la partie N-terminale du pro-peptide natriurétique de type B (Nt-proBNP) ou du BNP dans le sang du patient, et

ii) l'indicateur du métabolisme du calcium et/ou du phosphate sanguin est défini par la concentration de 25(OH)-vitamine $D_3$ dans le sang du patient.

4. Procédé selon la revendication 1 à 3, dans lequel un score de risque simplifié $Y_1$
égal à 0 ou égal à 1 indique un faible risque de mortalité,
égal à 2 ou égal à 3 indique un risque modéré de mortalité,
supérieur ou égal à 4 indique un risque élevé de mortalité.

5. Procédé selon la revendication 1 à 4, dans lequel le score de risque simplifié $Y_1$ est inversement associé à l'utilisation d'acide acétylsalicylique, de bêtabloquant(s) et/ou d'inhibiteurs de la synthèse du cholestérol, de préférence d'inhibiteurs de la CSE et/ou de statines.

6. Procédé pour déterminer le risque de mortalité d'un patient souffrant de diabète sucré, comprenant les étapes consistant à

a) déterminer un ensemble de paramètres biologiques obtenus chez le patient, où l'ensemble de paramètres biologiques comprend les variables suivantes :

le sexe du patient,

l'âge du patient,

au moins un indicateur de la performance myocardique, où l'au moins un indicateur de la performance myocardique est défini par la concentration d'un ou plusieurs peptides natriurétiques sélectionnés parmi le peptide natriurétique auriculaire (ANP) et le peptide natriurétique cérébral (BNP) tel que le préproANP, le proANP, la partie N-terminale du pro-ANP (Nt-proANP), le peptide mi-régional pronatriurétique auriculaire (MR-proANP), l'ANP, le préproBNP, le proBNP, la partie N-terminale du pro-peptide natriurétique de type B (Nt-proBNP), ou le BNP, dans le sang du patient,

un indicateur de la performance rénale, où l'indicateur de la performance rénale est défini par la concentration de rénine dans le sang du patient,

la durée du diabète sucré chez le patient définie en années,

un indicateur du métabolisme des lipides, où l'indicateur du métabolisme des lipides est défini par l'activité de la phospholipase A2 associée aux lipoprotéines (Lp-PLA2) dans le sang du patient, et

un indicateur du métabolisme du calcium et/ou du phosphate sanguin, où l'indicateur du métabolisme du calcium et/ou du phosphate sanguin est défini par la concentration de vitamine $D_3$ dans le sang du patient ;

b) mettre le paramètre biologique en corrélation, en obtenant ainsi un score de risque, où le score de risque est indicatif de la mortalité toutes causes confondues du patient au cours des prochaines années, et où le score de risque est indicatif d'un traitement médical du patient afin de réduire le risque de mortalité toutes causes confondues du patient,

en outre **caractérisé en ce que** le score de risque est un score de risque pondéré $Y_2$ et les paramètres biologiques sont mis en corrélation en utilisant la formule suivante, en obtenant ainsi le score de risque pondéré $Y_2$ :

$$Y_2 = 0,56 * \text{variable } 1 + 0,22 * \text{variable } 2 + 1,95 * (\text{variable } 3 - 4,17) + 0,41 * (\text{variable } 4 - 5,97) + 0,26 * (\text{variable } 5 - 3,28) + 0,85 * (\text{variable } 6 - 6,15) - 0,52 * (\text{variable } 7 - 2,60)$$

dans laquelle

a) variable 1 est définie comme égale à 1 si le sexe du patient est masculin et comme égale à 0 si le sexe du patient est féminin,

b) variable 2 est définie par la valeur logarithmique de la durée du diabète sucré en années,

c) variable 3 est définie par la valeur logarithmique de l'âge du patient en années,

d) variable 4 est définie par la valeur logarithmique de la concentration de la partie N-terminale du pro-peptide natriurétique de type B (Nt-proBNP) dans le sang du patient en ng/l,

e) variable 5 est définie par la valeur logarithmique de la concentration de rénine dans le sang du patient en pg/ml,

f) variable 6 est définie par la valeur logarithmique de l'activité de la phospholipase A2 associée aux lipoprotéines (Lp-PLA2) dans le sang du patient en U/l, et

g) variable 7 est définie par la valeur logarithmique de la concentration de vitamine $D_3$ dans le sang du patient, de préférence de la concentration de 25(OH)-vitamine $D_3$ dans le sang du patient, en ng/l,

où toutes les valeurs logarithmiques sont calculées par rapport à la base e, et où toutes les valeurs numériques dans la formule permettant d'obtenir le score de risque pondéré $Y_2$ sont définies avec un écart de 50 %, de préférence avec un écart de 20 %, plus préférablement avec un écart de 10 %, et le plus préférablement avec un écart de 5 %.

7. Procédé selon la revendication 6, dans lequel un score de risque pondéré $Y_2$
dans la plage inférieure ou égale à 0 indique un faible risque de mortalité,
dans la plage de 0 à 1 indique un risque modéré de mortalité, et
dans la plage supérieure ou égale à 1 indique un risque élevé de mortalité.

8. Procédé selon les revendications 2 à 7, dans lequel le risque de mortalité cardiovasculaire du patient est provoqué par une maladie de l'arbre artériel, de préférence par l'athérosclérose et/ou la thrombose.

9. Procédé selon l'une quelconque des revendications précédentes, dans lequel le score de risque est indicatif de la mortalité toutes causes confondues, de préférence de la mortalité cardiovasculaire, au cours des 3 prochaines années, de préférence au cours des 5 prochaines années, plus préférablement au cours des 10 prochaines années.

10. Procédé selon l'une quelconque des revendications précédentes, dans lequel le traitement médical du patient est sous la forme d'une administration de médicaments, de préférence dans lequel les médicaments sont destinés à traiter l'hyperlipidémie, réguler la pression artérielle, modifier la fréquence cardiaque, et/ou pour obtenir un contrôle de la glycémie, et/ou pour inhiber la coagulation sanguine chez des patients diabétiques.

11. Procédé selon la revendication 10, dans lequel les médicaments sont destinés à réduire la concentration de cholestérol dans le sang du patient, destinés à réduire la pression artérielle du patient, et/ou destinés à réduire la concentration de glucose dans le sang du patient.

12. Procédé selon l'une quelconque des revendications précédentes, dans lequel le score de risque est indicatif d'un traitement médical de patient présentant un risque élevé de mortalité.

13. Produit de programme informatique pouvant être directement chargé dans la mémoire interne d'un ordinateur numérique, comprenant des parties de code logiciel pour implémenter le calcul du score de risque de mortalité selon l'une quelconque des revendications précédentes lorsque ledit produit est exécuté sur un ordinateur.

14. Produit de programme informatique selon la revendication 13, comprenant des parties de code logiciel pour implémenter le calcul du score de risque simplifié $Y_1$ tel que défini dans la revendication 1, et/ou du score de risque pondéré $Y_2$ tel que défini dans la revendication 6.

# Figure 1

# Figure 2

A

B

## Figure 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060019315 A

**Non-patent literature cited in the description**

- **SCHNABEL et al.** *Eur Heart J,* 2010, vol. 31, 3024-3031
- **DOWDY ; WEARDEN.** Statistics for Research. John Wiley & Sons, 1983
- **BONOW.** *Circulation,* 1996, vol. 93, 1946
- **SMITH.** *J Endocrinol,* 2000, vol. 167, 239
- **MUELLER.** *Clin Chem Lab Med,* 2004, vol. 42, 942
- **WU.** *Clin Chem,* 2004, vol. 50, 867
- **ALA-KOPSALA.** *Clin Chem,* 2004, vol. 50, 1576
- **SCHNABEL et al.** *Eur Heart J,* 2010, vol. 31, 2024-3031
- **SESHASAI SR ; KAPTOGE S et al.** Diabetes mellitus, fasting glucose, and risk of cause-specific death. *N Engl J Med,* 2011, vol. 364, 829-841
- **SCHRAMM TK ; GISLASON GH ; KOBER L et al.** Diabetes patients requiring glucose-lowering therapy and nondiabetics with a prior myocardial infarction carry the same cardiovascular risk: a population study of 3.3 million people. *Circulation,* 2008, vol. 117, 1945-1954
- **HAFFNER SM ; LEHTO S ; RONNEMAA T ; PYORALA K ; LAAKSO M.** Mortality from coronary heart disease in subjects with type 2 diabetes and in nondiabetic subjects with and without prior myocardial infarction. *N Engl J Med,* 1998, vol. 339, 229-234
- **BLOOMGARDEN ZT.** Diabetes and cardiovascular disease. *Diabetes Care,* 2011, vol. 34, e24-30
- **MORROW DA.** Cardiovascular risk prediction in patients with stable and unstable coronary heart disease. *Circulation,* 2010, vol. 121, 2681-2691
- **GOLIASCH G ; KLEBER ME ; RICHTER B et al.** Routinely available biomarkers improve prediction of long-term mortality in stable coronary artery disease: the Vienna and Ludwigshafen Coronary Artery Disease (VILCAD) risk score. *Eur Heart J,* 2012, vol. 33, 2282-2289

- **STEVENS RJ ; KOTHARI V ; ADLER AI ; STRATTON IM.** United Kingdom Prospective Diabetes Study G (2001) The UKPDS risk engine: a model for the risk of coronary heart disease in Type II diabetes (UKPDS 56). *Clin Sci (Lond),* 2001, vol. 101, 671-679
- **ROOS CJ ; QUAX PH ; JUKEMA JW.** Cardiovascular metabolic syndrome: mediators involved in the pathophysiology from obesity to coronary heart disease. *Biomark Med,* 2012, vol. 6, 35-52
- **WINKELMANN BR ; MARZ W ; BOEHM BO et al.** Rationale and design of the LURIC study--a resource for functional genomics, pharmacogenomics and long-term prognosis of cardiovascular disease. *Pharmacogenomics,* 2001, vol. 2, 1-73
- **SACKS DB ; ARNOLD M ; BAKRIS GL et al.** Guidelines and recommendations for laboratory analysis in the diagnosis and management of diabetes mellitus. *Diabetes Care,* 2011, vol. 34, e61-99
- **SILBERNAGEL G ; SOURIJ H ; GRAMMER TB et al.** Isolated post-challenge hyperglycaemia predicts increased cardiovascular mortality. *Atherosclerosis,* 2012, vol. 225, 194-199
- **SILBERNAGEL G ; ROSINGER S ; GRAMMER TB et al.** Duration of type 2 diabetes strongly predicts all-cause and cardiovascular mortality in people referred for coronary angiography. *Atherosclerosis,* 2012, vol. 221, 551-557
- **STAMLER J ; VACCARO O ; NEATON JD ; WENTWORTH D.** Diabetes, other risk factors, and 12-yr cardiovascular mortality for men screened in the Multiple Risk Factor Intervention Trial. *Diabetes Care,* 1993, vol. 16, 434-444
- **CHO E ; RIMM EB ; STAMPFER MJ ; WILLETT WC ; HU FB.** The impact of diabetes mellitus and prior myocardial infarction on mortality from all causes and from coronary heart disease in men. *J Am Coll Cardiol,* 2002, vol. 40, 954-960
- **BULUGAHAPITIYA U ; SIYAMBALAPITIYA S ; SITHOLE J ; IDRIS I.** Is diabetes a coronary risk equivalent? Systematic review and meta-analysis. *Diabet Med,* 2009, vol. 26, 142-148

- **WANNAMETHEE SG ; SHAPER AG ; WHINCUP PH ; LENNON L ; SATTAR N.** Impact of diabetes on cardiovascular disease risk and all-cause mortality in older men: influence of age at onset, diabetes duration, and established and novel risk factors. *Arch Intern Med,* 2011, vol. 171, 404-410
- **BRUN E ; NELSON RG ; BENNETT PH et al.** Diabetes duration and cause-specific mortality in the Verona Diabetes Study. *Diabetes Care,* 2000, vol. 23, 1119-1123
- **NELSON RG ; SIEVERS ML ; KNOWLER WC et al.** Low incidence of fatal coronary heart disease in Pima Indians despite high prevalence of non-insulin-dependent diabetes. *Circulation,* 1990, vol. 81, 987-995
- **DOUST JA ; GLASZIOU PP ; PIETRZAK E ; DOBSON AJ.** A systematic review of the diagnostic accuracy of natriuretic peptides for heart failure. *Arch Intern Med,* 2004, vol. 164, 1978-1984
- **OMLAND T ; PERSSON A ; NG L et al.** N-terminal pro-B-type natriuretic peptide and long-term mortality in acute coronary syndromes. *Circulation,* 2002, vol. 106, 2913-2918
- **DE LEMOS JA ; MORROW DA ; BENTLEY JH et al.** The prognostic value of B-type natriuretic peptide in patients with acute coronary syndromes. *N Engl J Med,* 2001, vol. 345, 1014-1021
- **VAN HATEREN KJ ; LANDMAN GW ; KLEEFSTRA N et al.** The Midregional Fragment of Pro-A-Type Natriuretic Peptide, Blood Pressure, and Mortality in a Prospective Cohort Study of Patients With Type 2 Diabetes (ZODIAC-25). *Diabetes Care,* 2012
- **BHALLA MA ; CHIANG A ; EPSHTEYN VA et al.** Prognostic role of B-type natriuretic peptide levels in patients with type 2 diabetes mellitus. *J Am Coll Cardiol,* 2004, vol. 44, 1047-1052
- **TARNOW L ; GALL MA ; HANSEN BV ; HOVIND P ; PARVING HH.** Plasma N-terminal pro-B-type natriuretic peptide and mortality in type 2 diabetes. *Diabetologia,* 2006, vol. 49, 2256-2262
- **ATLAS SA.** The renin-angiotensin aldosterone system: pathophysiological role and pharmacologic inhibition. *Journal of managed care pharmacy : JMCP,* 2007, vol. 13, 9-20
- **TOMASCHITZ A ; PILZ S ; RITZ E et al.** Associations of plasma renin with 10-year cardiovascular mortality, sudden cardiac death, and death due to heart failure. *Eur Heart J,* 2011, vol. 32, 2642-2649
- **VERMA S ; GUPTA M ; HOLMES DT et al.** Plasma renin activity predicts cardiovascular mortality in the Heart Outcomes Prevention Evaluation (HOPE) study. *Eur Heart J,* 2011, vol. 32, 2135-2142
- **PARIKH NI ; GONA P ; LARSON MG et al.** Plasma renin and risk of cardiovascular disease and mortality: the Framingham Heart Study. *Eur Heart J,* 2007, vol. 28, 2644-2652
- **WANG TJ ; GONA P ; LARSON MG et al.** Multiple biomarkers for the prediction of first major cardiovascular events and death. *N Engl J Med,* 2006, vol. 355, 2631-2639
- **PARVING HH ; BRENNER BM ; MCMURRAY JJ et al.** Cardiorenal end points in a trial of aliskiren for type 2 diabetes. *N Engl J Med,* 2012, vol. 367, 2204-2213
- **JOERGENSEN C ; REINHARD H ; SCHMEDES A et al.** Vitamin D levels and asymptomatic coronary artery disease in type 2 diabetic patients with elevated urinary albumin excretion rate. *Diabetes Care,* 2012, vol. 35, 168-172
- **THOMAS GN ; O HARTAIGH B ; BOSCH JA et al.** Vitamin D levels predict all-cause and cardiovascular disease mortality in subjects with the metabolic syndrome: the Ludwigshafen Risk and Cardiovascular Health (LURIC) Study. *Diabetes Care,* 2012, vol. 35, 1158-1164
- **WINKLER K ; WINKELMANN BR ; SCHARNAGL H et al.** Platelet-activating factor acetylhydrolase activity indicates angiographic coronary artery disease independently of systemic inflammation and other risk factors: the Ludwigshafen Risk and Cardiovascular Health Study. *Circulation,* 2005, vol. 111, 980-987
- **WILENSKY RL ; SHI Y ; MOHLER ER, 3RD et al.** Inhibition of lipoprotein-associated phospholipase A2 reduces complex coronary atherosclerotic plaque development. *Nat Med,* 2008, vol. 14, 1059-1066
- **LP PLASC ; THOMPSON A ; GAO P et al.** Lipoprotein-associated phospholipase A(2) and risk of coronary disease, stroke, and mortality: collaborative analysis of 32 prospective studies. *Lancet,* 2010, vol. 375, 1536-1544
- **HATOUM IJ ; HU FB ; NELSON JJ ; RIMM EB.** Lipoprotein-associated phospholipase A2 activity and incident coronary heart disease among men and women with type 2 diabetes. *Diabetes,* 2010, vol. 59, 1239-1243
- **WANG TJ.** Multiple biomarkers for predicting cardiovascular events: lessons learned. *J Am Coll Cardiol,* 2010, vol. 55, 2092-2095
- **CHAMNAN P ; SIMMONS RK ; SHARP SJ ; GRIFFIN SJ ; WAREHAM NJ.** Cardiovascular risk assessment scores for people with diabetes: a systematic review. *Diabetologia,* 2009, vol. 52, 2001-2014
- **WANG KL ; LIU CJ ; CHAO TF et al.** Statins, risk of diabetes, and implications on outcomes in the general population. *J Am Coll Cardiol,* 2012, vol. 60, 1231-1238
- **NICOLUCCI A ; STANDL E.** Antiplatelet therapy for every diabetic person?. *Diabetes Care,* 2011, vol. 34 (2), 150-154